# EUROPEAN PATENT APPLICATION

(11) **EP 3 564 263 A1**
(43) Date of publication of application: **06.11.2019**
(21) Application number: 18170659.9
(22) Date of filing: 03.05.2018
(51) Int. Cl.: C07K 16/08, C07K 16/28, C12N 15/63, A61K 39/00

(54) **FUSION PROTEINS COMPRISING A CELL SURFACE MARKER SPECIFIC VHH**

(71) Applicant: Universitätsklinikum Hamburg-Eppendorf, 20246 Hamburg (DE)
(72) Inventor: NOLTE, Friedrich, 20457 Hamburg (DE); EICHHOFF, Anna Marei, 20253 Hamburg (DE)
(74) Representative: Maiwald Patent- und Rechtsanwaltsgesellschaft mbH

(57) **Abstract**

The present invention relates to fusion proteins comprising a cell surface marker specific VHH. In particular, the invention relates to bispecific VHH adaptor proteins, i.e. fusion proteins comprising an adeno-associated virus (AAV) specific VHH linked to a cell surface marker specific VHH. Moreover, the invention relates to a recombinant AAV which comprises a fusion protein in which a cell surface marker specific VHH is integrated into a capsid protein of the AAV. Also nucleotide sequences encoding such fusion proteins, vectors are contemplated. The invention moreover refers to uses of the fusion proteins and nucleic sequences for gene therapy.

## Description

### FIELD OF THE INVENTION

The present invention relates to fusion proteins comprising a cell surface marker specific variable heavy chain domain of an heavy chain antibody (VHH). In particular, the invention relates to bispecific VHH adaptor proteins, i.e. fusion proteins comprising an adeno-associated virus (AAV) specific VVH linked to a cell surface marker specific VHH. Moreover the invention relates to a recombinant AAV which comprises a fusion protein in which a cell surface marker specific VHH is integrated into a capsid protein of the AAV. Also nucleotide sequences encoding such fusion proteins, vectors are contemplated. The invention moreover refers to uses of the fusion proteins and nucleic sequences for gene therapy.

### BACKGROUND OF THE INVENTION

Adeno-associated viruses (AAVs) are of increasing importance for gene and tumor therapy. AAVs are used as vectors, since they are non-pathogenic, show low immunogenicity, and confer long-term expression of the transferred gene.

A challenge in the use of AAVs as vectors in gene therapy is their broad tropism, i.e. the parallel infection of several tissues and cell types. Moreover, neutralizing antibodies that occur in about 80 % of the population impede the efficient binding and transduction of target cells.
Current techniques of improving target specificity of AAVs include the chemical conjugation of two Fab fragments (Bartlett et al. 1999), the insertion of peptides into the capsid proteins, the fusion of DARPins to the N-terminus of the VP2 capsid protein (Münch et al. 2013), the use of AAV serotypes that are not recognized by neutralizing antibodies.

However, AAVs produced by these strategies still only show insufficient transduction efficiency of AAV vectors due to inefficient uptake, uncoating and/or translocation of the viral genome to the nucleus of the host cell.

Thus, there is a need to improve AAVs for gene therapy, in particular to improve tissue specificity of AAVs.

### OBJECTIVES AND SUMMARY OF THE INVENTION

The present invention solves this problem by using cell surface maker specific variable heavy chain domains of heavy chain antibodies (VHH), in particular fusion proteins comprising cell surface marker specific VHHs for improving the cell specificity of AAVs. In particular, two strategies are described herein: Bispecific adaptor proteins that bind to AAVs and to specific cell surface markers are used to enhance the transduction of cells expressing the respective cell surface markers. Further, cell surface marker specific VHHs are integrated directly into AAVs via fusion proteins of the AAV capsid proteins with the cell surface marker specific VHH domain.

Accordingly, a first aspect of the invention refers to a fusion protein comprising a cell surface marker specific VHH.

In some embodiments the fusion protein comprises a cell surface marker specific VHH and an adeno-associated virus (AAV) specific VHH.

The serotype of the AAV may be selected from the group consisting of AAV1, AAV2, AAV3, AAV4, AAV5, AAV6, AAV7, AAV8, AAV9, AAV10, AAV11, AAV12, AAVrh10 or variants thereof or a combination thereof. Preferably, the serotype of the AAV is AAV1 and/or AAV2 or variants thereof.

A specific embodiment refers to bispecific VHH adaptors that bind to the AAV1 serotype. Exemplary sequences of the AAV1 specific VHH are set out in SEQ ID NO: 20 to 24 or sequences having at least 80% identity thereto.

A specific embodiment refers to bispecific VHH adaptors that bind to the AAV1 and AAV2 serotype. Exemplary sequences of the AAV1/2 dual specific VHH are set out in SEQ ID NO: 25 to 27 or sequences having at least 80% identity thereto.

Typically, the cell surface marker is a membrane protein or a membrane glycolipid. The membrane protein may be selected, for example, from the group consisting of multi-span homo trimer membrane proteins, such as P2X7, type-II single span membrane proteins, such as CD38, GPI-anchor membrane proteins, such as ARTC2.2, integrins, type I membrane proteins, such as members of the immunoglobulin superfamily, receptors, such as cytokine and growth factor receptors or G-protein coupled receptors.

One embodiment refers to a bispecific VHH adaptor, i.e. a fusion protein that comprises a VHH specific for the membrane protein P2X7 as well as a VHH specific for the AAV1 serotype. In one embodiment the protein sequence of such a fusion protein is set out in SEQ ID NO:4 or is at least 80% identical thereto.

One embodiment refers to a bispecific VHH adaptor, i.e. a fusion protein that comprises a VHH specific for the membrane protein P2X7 as well as an AAV1/2 dual specific VHH. In one embodiment the protein sequence of such a fusion protein is set out in SEQ ID NO:5 or is at least 80% identical thereto.

One embodiment refers to a bispecific VHH adaptor, i.e. a fusion protein that comprises a VHH specific for the membrane protein CD38 as well as a VHH specific for the AAV1 serotype. In one embodiment the protein sequence of such a fusion protein is set out in SEQ ID NO:6 or is at least 80% identical thereto.

One embodiment refers to a bispecific VHH adaptor, i.e. a fusion protein that comprises a VHH specific for the membrane protein CD38 as well as an AAV1/2 dual specific VHH. In one embodiment the protein sequence of such a fusion protein is set out in SEQ ID NO:7 or is at least 80% identical thereto.

One embodiment refers to a bispecific VHH adaptor, i.e. a fusion protein that comprises a VHH specific for the membrane protein ARTC2.2 as well as a VHH specific for the AAV1 serotype. In one embodiment the protein sequence of such a fusion protein is set out in SEQ ID NO:8 or is at least 80% identical thereto.

One embodiment refers to a bispecific VHH adaptor, i.e. a fusion protein that comprises a VHH specific for the membrane protein ARTC2.2 as well as an AAV1/2 dual specific VHH. In one embodiment the protein sequence of such a fusion protein is set out in SEQ ID NO:9 or is at least 80% identical thereto.

Typically, the fusion protein comprises a linker between the cell surface marker specific VHH and the VHH specific for AAV. The linker may be a peptide linker, such as the linker sequence set out in SEQ ID NO: 19.

Another embodiment refers to a recombinant AAV capsid protein, i.e. a fusion protein comprising a cell surface marker specific VHH and an adeno-associated virus (AAV) capsid protein.

The serotype of the AAV may be selected from the group consisting of AAV1, AAV2, AAV3, AAV4, AAV5, AAV6, AAV7, AAV8, AAV9, AAV10, AAV11, AAV12, AAVrh10 or variants thereof or a combination thereof. Preferably, the serotype of the AAV is AAV1 and/or AAV2 or variants thereof.

In some embodiments, the AAV capsid protein in which the cell surface marker specific VHH is integrated is VP1 or VP2. In a preferred embodiment the AAV capsid protein is VP1.

The cell surface marker specific VHH may be directed against any membrane protein or membrane glycolipid capable of binding a VHH.

Some embodiments refer to an AAV capsid protein, in which a VHH specific for P2X7 is integrated. In a specific embodiment the AAV is of the AAV2 serotype. An exemplary sequence for such a protein is set out in SEQ ID NO:1 or is at least 80% identical thereto.

Some embodiments refer to an AAV capsid protein, in which a VHH specific for CD38 is integrated. In a specific embodiment the AAV is of the AAV2 serotype. An exemplary sequence for such a protein is set out in SEQ ID NO:2 or is at least 80% identical thereto.

Some embodiments refer to an AAV capsid protein, in which a VHH specific for ARTC2.2 is integrated. In a specific embodiment the AAV is of the AAV2 serotype. An exemplary sequence for such a protein is set out in SEQ ID NO:3 or is at least 80% identical thereto.

Another aspect of the invention refers to a nucleotide sequence encoding the fusion protein as described above. Also contemplated are vectors comprising such a nucleotide sequence.

A further aspect refers to an AAV containing at least one of the fusion proteins as described above.

The invention also refers to the use of the fusion proteins, the nucleotide sequence according, the vector and the AAV as described herein for improving the transduction efficiency of the AAV targeted cells.

The invention also refers to the use of the fusion proteins, the nucleotide sequence according, the vector and the AAV as described herein for introducing a defined nucleic sequence into a target cell.

A further aspect of the invention relates to the fusion proteins the nucleotide sequences, the vectors, the AAVs as described herein for use as a medicament.

Thus, one embodiment refer to the fusion proteins the nucleotide sequences, the vectors, the AAVs as described herein for use in gene therapy.

In particular, one embodiment refer to the fusion proteins the nucleotide sequences, the vectors, the AAVs as described herein for use in the treatment of cancer.

### FIGURE LEGENDS

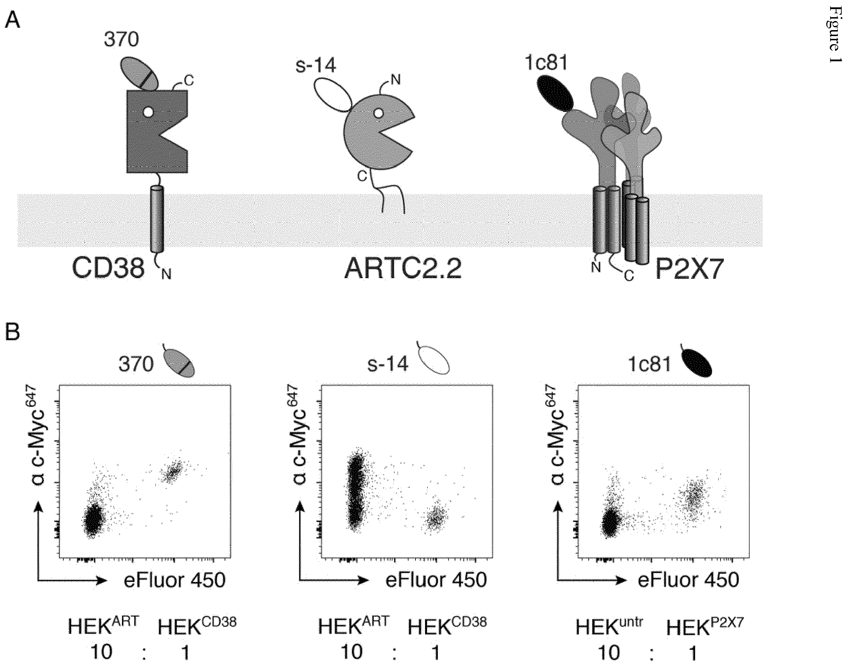

**Figure 1****. Membrane proteins and nanobodies used for retargeting of AAV**
   **A**) Human CD38 is a single-span type II transmembrane protein carrying a C-terminal ecto-enzyme domain. Mouse ARTC2.2 is a GPI-anchored raft-associated ecto-enzyme. Mouse P2X7 is a homotrimeric ion channel that is gated by extracellular ATP or by ADP-ribosylation. Each subunit carries two transmembrane domains with cytosolic N-and C-termini. CD38-, ARTC2.2- and P2X7-specific nanobodies (MU370, s-14 and 1c81, respectively) used here for specific targeting of AAV to these membrane proteins were previously selected in the Nolte lab from immunized llamas (Fumey et al 2017, Koch-Nolte et al 2007, Danquah et al 2016).
   **B**) Flow cytometry analyses of P2X7, CD38 and ARTC2.2 expression by stably transfected HEK cells.

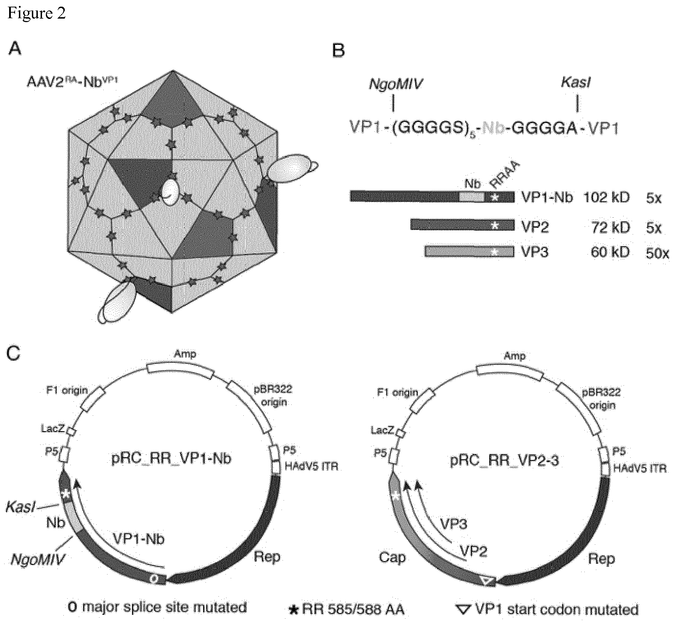

**Figure 2****. Genetic insertion of a membrane protein-specific nanobody into AAV2^{RA}**
   **A**) Schematic of the modified AAV2 capsid. AAV capsids consist of 60 capsid proteins (∼50 VP3, ∼5 VP2, ∼5 VP1). Two arginine residues (R585, R588) that mediate binding of AAV2 to HSPG were mutated to alanine (indicated by stars and the superscript "RA": AAV2^{RA}). The nanobody was inserted via peptide linkers into the exposed surface loop of VP1 at position 453-459 (indicated by "-Nb^{VP1}").
   **B**) Schematic of the Nanobody insertion site. The insertion site is flanked on the 5' (N-terminal) side by a NgoMIV restriction site and a 25 amino acid GGGGS₅ linker peptide, and on the 3' (C-terminal) side by a short GGGGA linker and a KasI restriction site.
   C) Two plasmids were used in order to force assembly of virions containing the VP1-Nb fusion: pRC_RR_VP1-Nb encodes the VP1-Nb fusion protein, expression of VP2 and VP3 from this plasmid is inhibited by mutation of the corresponding splice site. pRC_RR_VP2-3 encodes VP2 and VP3, expression of VP1 from this plasmid is inhibited by mutation of the VP1 start codon.

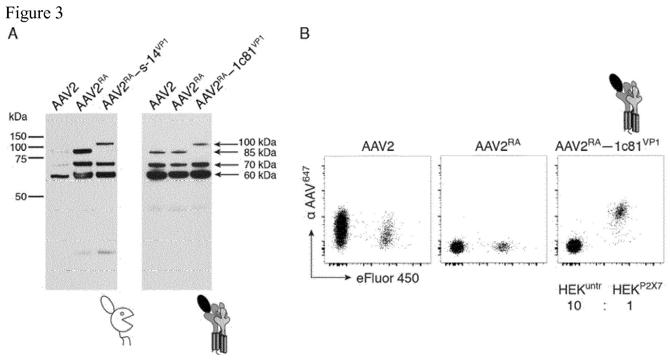

**Figure 3****: Nb-VP1 fusion proteins are assembled into AAV2^{RA} and mediate specific binding of nanobody-modified virions to target-expressing HEK cells.**
   **A)** AAV were purified from transfected HEK cells and subjected to Western-Blot analyses with mAb B1 that recognizes a common epitope near the C-terminus of all capsid proteins. The three capsid proteins are assembled at a ratio of ∼ 50 VP3 (60 kDa) : 5 VP2 (70 kDa) : 5 VP1 (85 kDa). As expected, the VP1-Nb fusion migrates at a higher Mr (100 kDa) than VP1. s-14 = ARTC2.2-specific nanobody, 1c81 = P2X7-specific nanobody.
   **B)** Untransfected HEK cells (HEK^{untr}) and eFluor 450-labeled, P2X7-transfected HEK cells (HEK^{P2X7}) were mixed at a ratio of ∼10:1 and assessed for binding of AAV2, AAV2^{RA}, and AAV2^{RA}-1C81^{VP1} by flow cytometry. Cells were incubated for 30 min at 4°C with the indicated virions and washed 2 x with PBS. Bound virions were detected with an Alexa647-conjugated AAV2-specific recombinant antibody (scFv A20-rbFc).

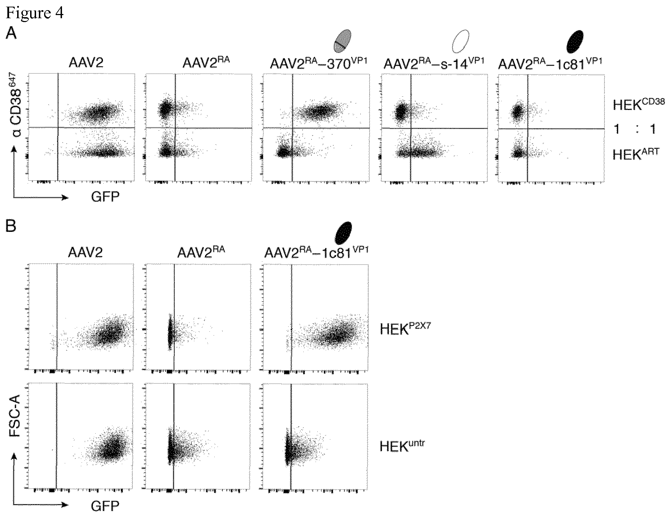

**Figure 4****: Nanobody-VP1 fusion proteins mediate specific transduction of target-expressing HEK cells**
   Untransfected HEK cells (HEK^{untr}) and HEK cells stably transfected with ARTC2.2, CD38, or P2X7 (HEK^{ART}, HEK^{CD38} and HEK^{P2X7}, respectively) were seeded in 24 well plates either as (**A**) a 1:1 mixture (HEK^{ART} + HEK^{CD38}) or (**B**) separately (HEK^{untr}, HEK^{P2X7}) before addition of GFP-encoding AAV2, AAV2^{RA}, or AAV2^{RA}-Nb^{VP1}. 48 h after addition of GFP-encoding virions, transduction efficiency was assessed by measuring cellular GFP-fluorescence by flow cytometry. In order to distinguish co-cultured HEK^{ART} from HEK^{CD38} cells in (**A**), cells were incubated with an Alexa647-conjugated CD38-specific recombinant antibody (JK36-rbFc) immediately before analysis.

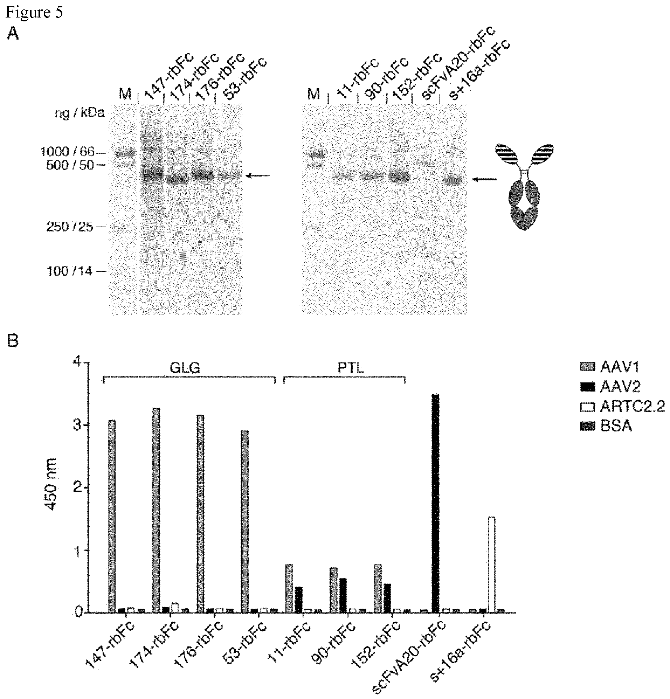

**Figure 5****. Selection of AAV1-specific and of AAV1-AAV2 dual-specific nanobodies.**
   **A**) Lama-IgH-transgenic mice were immunized with AAV1 and AAV2. RNA obtained from lymph nodes and spleen three days after the last boost immunization was transcribed into cDNA. The VHH repertoire was PCR amplified and cloned into the pCSE2.5 vector, thereby fusing the coding sequences of the Ig kappa signal peptide, the VHH domain, and the hinge, CH2 and CH3 domains of rabbit IgG. Secreted nanobody-rabbit IgG heavy chain antibodies were produced in transfected HEK-6E cells. Proteins in supernatants harvested 6 days after transfection were analyzed by SDS-PAGE (under reducing conditions) and Coomassie staining. The prominent band at ∼45 kDa (arrows) corresponds to the nanobody-rabbit Fc fusion protein. The single chain variable fragment (scFv) of AAV2-specific mAb A20 and the ARTC2.2-specific nanobody s+16a were similarly expressed as rabbit IgG heavy chain antibodies. As expected, the scFv-rbFc fusion protein displays a higher Mr of ∼ 60 kDa.
   **B**) Binding of nanobody/scFv-rabbit heavy chain antibodies to AAV1, AAV2, ARTC2.2 and BSA was assessed by ELISA. Wells were coated with 1 x 10⁹ vg or 100 ng of purified proteins and free binding sites were blocked with BSA. Bound antibodies were detected with horse radish peroxidase-conjugated, rabbit IgG-specific antibodies and TMB as substrate.

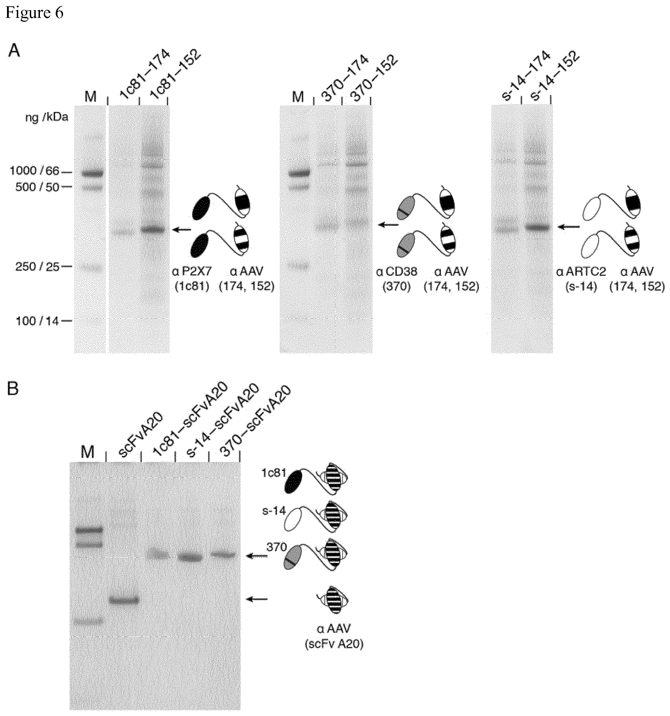

**Figure 6****. Production of bispecific nanobody adaptors.**
   **A**) Schematic of bispecific nanobody adaptors. A membrane protein specific nanobody (1c81, 370, or s-14) was genetically fused via a 25 amino acid GGGGS₅ peptide linker to an AAV-specific nanobody (174, 152) or scFv (A20). These bispecific nanobody adaptors were cloned into the pCSE2.5 vector so as to fuse the coding sequences of the Ig kappa signal peptide, the nanobody-adaptor, and a chimeric His6x-c-myc tag. Secreted nanobody-adaptors were produced in transfected HEK-6E cells, purified by immobilized metal affinity chromatography, and analyzed by SDS-PAGE and Coomassie staining. The prominent band at ∼30 kDa (arrows) corresponds to the nanobody-dimers. As expected the scFv A20-containing adaptors migrate at a higer Mr of ∼ 45 kDa.

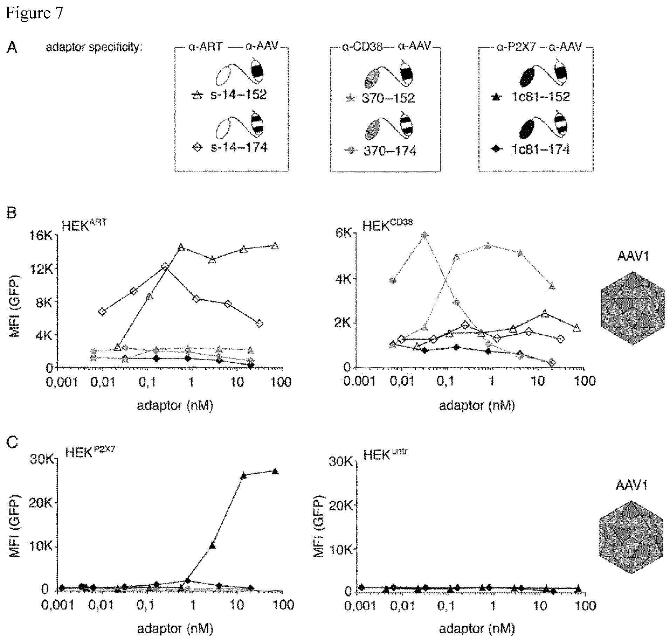

**Figure 7****. Bispecific nanobody adaptors mediate specific transduction of target-expressing HEK cells with AAV1.**
   Untransfected HEK cells (HEK^{untr}) and HEK cells stably transfected with ARTC2.2, CD38, or P2X7 (HEK^{ART}, HEK^{CD38} and HEK^{P2X7}, respectively) were seeded in 96 well plates either as (**A**) a 1:1 mixture (HEK^{ART} + HEK^{CD38}) or (**B**) separately (HEK^{untr}, HEK^{P2X7}) before addition of GFP-encoding AAV1 and titrated nanobody adaptors. 48 h after addition of virions, transduction efficiency was assessed by measuring GFP-fluorescence by flow cytometry. In order to distinguish co-cultured HEK^{ART} from HEK^{CD38} cells in (**A**), the HEK^{CD38} cells were loaded with eFluor450 before seeding.

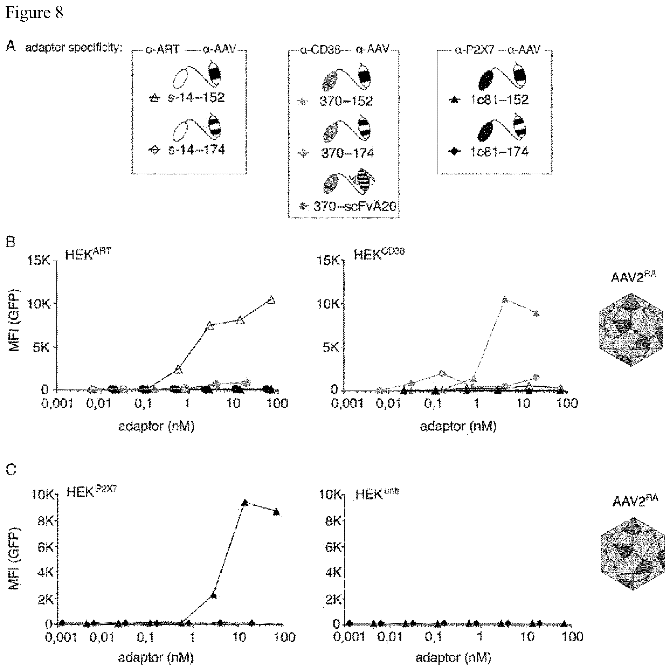

**Figure 8****. Bispecific nanobody adaptors mediate specific transduction of target-expressing HEK cells with AAV2^{RA}.**
   Untransfected HEK cells (HEK^{untr}) and HEK cells stably transfected with ARTC2.2, CD38, or P2X7 (HEK^{ART}, HEK^{CD38} and HEK^{P2X7}, respectively) were seeded in 96 well plates either as (**A**) a 1:1 mixture (HEK^{ART} + HEK^{CD38}) or (**B**) separately (HEK^{untr}, HEK^{P2X7}) before addition of GFP-encoding AAV2^{RA} and titrated nanobody adaptors. 48 h after addition of virions, transduction efficiency was assessed by measuring GFP-fluorescence by flow cytometry. In order to distinguish co-cultured HEK^{ART} from HEK^{CD38} cells in (**A**), the HEK^{CD38} cells were loaded with eFluor450 before seeding.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

In order to specifically target vehicles, such as AAVs to a specific cell population the invention provides a fusion protein comprising a cell surface marker specific variable heavy chain of a heavy chain antibody (VHH).

The term "fusion protein" denotes proteins in which at least two polypeptides that originally coded for separate proteins or protein domains are combined in a single molecule. The at least two polypeptides are typically connected by translation of the coding sequence in a cell or cell-free extract into a single polypeptide.

Llamas carry a variant immunoglobulin locus that permits the generation of unusual antibodies composed only of heavy chains. The single variable domain of these antibodies (designated VHH, sdAb, or nanobody) is the smallest antigen-binding domain generated by adaptive immune systems. With a long complementarity-determining region 3 (CDR3), VHHs can extend into crevices on proteins that are not accessible to conventional antibodies, including functionally interesting sites such as the active site of an enzyme or the receptor-binding canyon on a virus surface. VHHs offer numerous other advantages compared to conventional antibodies carrying variable domains (VH and VL) of conventional antibodies, including higher stability, solubility, expression yields, and refolding capacity, as well as better in vivo tissue penetration. Since VHH do not bind to VL domains, it is much easier to reformat VHHs into bispecific antibody constructs than constructs containing conventional VH-VL pairs or single domains based on VH domains.

In one embodiment the fusion protein further comprises an adeno-associated virus (AAV) specific VHH. Such a fusion protein including a cell surface marker specific VHH and a AAV specific VHH is also termed bispecific VHH adaptor protein. These proteins can increase the tissue specificity and/or target cell specificity of the AAV and thereby increase the transduction efficiency of the AAVs.

AAV are small non-enveloped viruses which belong to the family of *Parvoviridae.* The AAV genome consists of single stranded DNA of about 4.7 kilo bases. It comprises T-shaped inverted terminal repeats (ITRs) of 145 bases on each end and carries two open reading frames (ORF): rep and cap, which encode for functional and capsid proteins. The ORF rep encodes for four Rep proteins (Rep78, Rep68, Rep52 and Rep40). Rep 78 and Rep 68 have site-specific, single-strand endonuclease, DNA helicase, and ATP activities, respectively, which are responsible for DNA replication. Rep52 and Rep40 are responsible for packaging of DNA flanked by ITRs into capsids. The ORF cap encodes for three capsid proteins: VP1, VP2 and VP3. Additionally, the VP1 mRNA encodes for the assembly-activating protein (AAP) in a different reading frame. Based in the differences in the amino acid sequences of the capsid proteins, different AAV serotypes are distinguished, i.e. AAV1, AAV2, AAV3, AAV4, AAV5, AAV6, AAV7, AAV8, AAV9, AAV10, AAV11, AAV12, AAVrh10.

Thus, in a specific embodiment the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof.

An exemplary variant is AAV2RA, wherein AAV2 is modified in R585 and R588 mediating binding of AAV2 to HSPG and thereby the binding to HSPG is inhibited (Boucas et al. 2009; Münch et al. 2013).

The skilled person is also aware of other variants, which are for example modified by the insertion of peptides in the region containing R585 and R588. Other variations are chimeric AAVs, in which the capsid proteins stem from different (e.g. VP1 stemming from AAV and VP2 und VP3 stemming from AAV2), or AAVs in which all capsid proteins contain sequence sections stemming from different AAV serotypes.
In a preferred embodiment the serotype of the AAV is AAV1 and/or AAV2 or variants thereof.

A specific embodiment refers to bispecific VHH adaptors that bind to the AAV1 serotype. Exemplary sequences of the AAV1 specific VHH are set out in SEQ ID NO: 20 to 24 or sequences having at least 80% identity thereto.

A specific embodiment refers to bispecific VHH adaptors that bind to the AAV1 and AAV2 serotypes. Exemplary sequences of the AAV1/AAV2 dual-specific VHH are set out in SEQ ID NO: 25 to 27 or sequences having at least 80% identity thereto.

Some embodiments refer to bispecific VHH adaptors that bind to the AAV1 serotype, wherein these fusion proteins comprise a AAV1 specific VHH having a CDR3 domain which is selected from the sequences set out in SEQ ID NO: 28 to 30 or sequences having at least 80% identity thereto.

A specific embodiment refers to bispecific VHH adaptors that bind to the AAV1 and AAV2 serotype. Exemplary sequences of the AAV1/AAV2 dual-specific VHH having a CDR3 domain which is set out in SEQ ID NO: 31 or sequences having at least 80% identity thereto.

In a specific embodiment, the cell surface marker is a membrane protein.

The term "cell surface marker" refers to proteins or carbohydrates that are present on the surface of a cell.

The term "membrane protein" refers to proteins that are attached or integrated into the membrane of a cell. The term includes integral membrane proteins being permanently anchored to the membrane and peripheral membrane proteins that are temporarily attached to the lipid bilayer or to integral membrane proteins thereof. The integral membrane proteins may have one or several transmembrane domains. The peripheral membrane proteins may be integrated to the membrane via one or several α-helixes, one or several hydrophobic loops, may be covalently bound to a membrane lipid or interact by electrostatic or ionic interactions with membrane lipids or transmembrane proteins. The membrane protein may be selected from the group consisting of multi-span homo trimer membrane protein, type-II single span membrane protein, GPI-anchor membrane protein, integrins, type I membrane proteins, such as members of the immunoglobulin superfamily, receptors, such as cytokine and growth factor receptors or G-protein coupled receptors.

The term "carbohydrate" particularly refers to glycolipids.

The cell surface marker specific VHH may be directed against any membrane protein or membrane glycolipid capable of binding a VHH.

In one embodiment the multi-span trimer membrane protein is P2X7. P2X7 is prominently expressed by monocytes and regulatory T cells and is overexpressed on the surface of many tumor cells.

The type-II single span membrane protein may be for example CD38. CD38 is prominently expressed by plasma cells and NK cells. CD38 is overexpressed on the surface of many tumor cells.

An exemplary glycosyl-phosphatidylinositol (GPI) -anchor membrane protein is ARTC2.2.

In a specific embodiment, the membrane protein is P2X7 and the AAV is of the AAV1 serotype. The protein sequence of such a fusion protein is set out in SEQ ID NO:4. Also proteins having a sequence which is at least 80% identical to SEQ ID NO: 4 are encompassed. For example, the protein sequence may be at least 70%, at least 80 %, at least 90 %, at least 95%, at least 97 % identical to the sequence set out in SEQ ID NO:4.

In another specific embodiment, the membrane protein is P2X7 and the AAV is of the AAV1 or AAV2 serotype. In other words the fusion protein comprises a VHH specific for the membrane protein P2X7 as well as an AAV1/2 dual specific VHH. The protein sequence of such a fusion protein is set out in SEQ ID NO:5. Also proteins having a sequence which is at least 80% identical to SEQ ID NO: 5 are encompassed. For example, the protein sequence may be at least 70%, at least 80 %, at least 90 %, at least 95%, at least 97 % identical to the sequence set out in SEQ ID NO:5.

In a further specific embodiment, the membrane protein is CD38 and the AAV is of the AAV1 serotype. The protein sequence of such a fusion protein is set out in SEQ ID NO:6. Also proteins having a sequence which is at least 80% identical to SEQ ID NO: 6 are encompassed. For example, the protein sequence may be at least 70%, at least 80 %, at least 90 %, at least 95%, at least 97 % identical to the sequence set out in SEQ ID NO:6.

In a further embodiment, the membrane protein is CD38 and the AAV is of the AAV1 or AAV2 serotype. In other words the fusion protein comprises a VHH specific for the membrane protein CD38 as well as an AAV1/2 dual specific VHH. The protein sequence of such a fusion protein is set out in SEQ ID NO:7. Also proteins having a sequence which is at least 80% identical to SEQ ID NO: 7 are encompassed. For example, the protein sequence may be at least 70%, at least 80 %, at least 90 %, at least 95%, at least 97 % identical to the sequence set out in SEQ ID NO:7.

In a further embodiment, the membrane protein is ARTC2.2 and the AAV is of the AAV1 serotype. The protein sequence of such a fusion protein is set out in SEQ ID NO:8. Also proteins having a sequence which is at least 80% identical to SEQ ID NO:8 are encompassed. For example, the protein sequence may be at least 70%, at least 80 %, at least 90 %, at least 95%, at least 97 % identical to the sequence set out in SEQ ID NO:8.

In another embodiment, the membrane protein is ARTC2.2 and the AAV is of the AAV1 or AAV2 serotype. In other words the fusion protein comprises a VHH specific for the membrane protein ARTC2.2 as well as an AAV1/2 dual specific VHH. The protein sequence of such a fusion protein is set out in SEQ ID NO:9. Also proteins having a sequence which is at least 80% identical to SEQ ID NO: 9 are encompassed. For example, the protein sequence may be at least 70%, at least 80 %, at least 90 %, at least 95%, at least 97 % identical to the sequence set out in SEQ ID NO:9.

The determination of percent identity between multiple sequences is preferably accomplished using the AlignX application of the Vector NTI AdvanceTM 10 program (Invitrogen Corporation, Carlsbad CA, USA). This program uses a modified Clustal W algorithm (Thompson et al., 1994. Nucl Acids Res. 22: pp. 4673-4680; Invitrogen Corporation; Vector NTI AdvanceTM 10 DNA and protein sequence analysis software. User's Manual, 2004, pp.389-662). The determination of percent identity is performed with the standard parameters of the AlignX application.

Typically, the fusion protein comprises a linker between the cell surface marker specific VHH and the VHH specific for AAV. The skilled person is aware of linkers that are suitable for combining two functional polypeptides (Els Conrath et al. 2001).

In a preferred embodiment the linker is a peptide linker. The peptide linker may be for example 25 amino acid glycine/serine linker. In one embodiment, the sequence of the peptide linker is set out in SEQ ID NO: 19.

### Recombinant nanobody-AAV

One embodiment of the invention refers to a fusion protein comprising a cell surface marker specific VHH and an adeno-associated virus (AAV) capsid protein. In such embodiments the VHH conveying the target cell specificity is directly integrated into AAV via insertion of the VHH protein sequence into the AAV sequence.

The term "target cell" as used herein, refers to a specific cell type or to a group of specific cell types. The target cell may be of endodermal, ectodermal or mesodermal origin. The cell of endodermal origin may be exocrine secretory epithelial cells or a hormone secreting cell. The cell of ectodermal origin may be a cell of the integumentary system or the nervous system. The cell of the mesodermal origin may be a metabolism or storage cell, such as adipocytes, a barrier function cell, such as a lung cell, gut, exocrine glands and urogenital tract cells; extracellular matrix cells, contractile cells, blood and immune system cells, germ cells, nurse cells or interstitial cells. The target cells may also be for example monocytes, T cells or natural killer cells. The target cell may be a tumor cell.

Thus, in a specific embodiment the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof.

In a preferred embodiment the serotype of the AAV is AAV1 and/or AAV2 or variants thereof.

Typically, the VHH is integrated into AAV capsid protein VP2 or VP1 in order to incorporate approximately VHH into the capsid. During assembly, VP1, VP2, and VP3 are incorporated at a ratio of 1:1:10 into AAV capsids. Preferably the VVH is integrated into VP1 . VP1 - in contrast to VP2 - is required for efficient infection due to the N-terminal phospholipase A2 domain (Girod et al. 2002; Warrington et al. 2004). The fact that VP1 is encoded by a separate messenger RNA, while VP2 and VP3 are encoded by a common mRNA, facilitates the assembly of VHH-containing chimeric capsids of different AAV serotypes.

The cell surface marker may be membrane protein. In a specific embodiment, the membrane protein may be selected from the group consisting of multi-span homo trimer membrane proteins, such as P2X7, type-II single span membrane proteins, such as CD38, GPI-anchor membrane proteins, such as ARTC2.2, integrins, type I membrane proteins, such as members of the immunoglobulin superfamily, receptors, such as cytokine and growth factor receptors or G-protein coupled receptors.

In a specific embodiment, the membrane protein is P2X7 and the AAV is of the AAV2 serotype. The protein sequence of such recombinant AAV is set out in SEQ ID NO:1. Also proteins having a sequence which is at least 80% identical to SEQ ID NO: 1 are encompassed. For example, the protein sequence may be at least 70%, at least 80 %, at least 90 %, at least 95%, at least 97 % identical to the sequence set out in SEQ ID NO:1.

In another specific embodiment the membrane protein is CD38 and the AAV is of the AAV2 serotype. The protein sequence of such recombinant AAV is set out in SEQ ID NO:2. Also proteins having a sequence which is at least 80% identical to SEQ ID NO: 2 are encompassed. For example, the protein sequence may be at least 70%, at least 80 %, at least 90 %, at least 95%, at least 97 % identical to the sequence set out in SEQ ID NO:2.

In a further specific embodiment, the membrane protein is ARTC2.2 and the AAV is of the AAV2 serotype. The protein sequence of such recombinant AAV is set out in SEQ ID NO:3. Also proteins having a sequence which is at least 80% identical to SEQ ID NO: 3 are encompassed. For example, the protein sequence may be at least 70%, at least 80 %, at least 90 %, at least 95%, at least 97 % identical to the sequence set out in SEQ ID NO:3.

In some embodiments, the amino acid sequence of the fusion protein may comprise one or more phenotypically silent substitutions.

"Phenotypically silent substitutions" are also named "conservative amino acid substitutions". The concept of "conservative amino acid substitutions" is understood by the skilled artisan, and preferably means that codons encoding positively-charged residues (H, K, and R) are substituted with codons encoding positively-charged residues, codons encoding negatively- charged residues (D and E) are substituted with codons encoding negatively-charged residues, codons encoding neutral polar residues (C, G, N, Q, S, T, and Y) are substituted with codons encoding neutral polar residues, and codons encoding neutral non-polar residues (A, F, I, L, M, P, V, and W) are substituted with codons encoding neutral non-polar residues. These variations can spontaneously occur, be introduced by random mutagenesis, or can be introduced by directed mutagenesis. Those changes can be made without destroying the essential characteristics of these polypeptides. The ordinarily skilled artisan can readily and routinely screen variant amino acids and/or the nucleic acids encoding them to determine if these variations substantially reduce or destroy the ligand binding capacity by methods known in the art.

The skilled person understands, that also the nucleic acid encoding the fusion protein may be modified. Useful modifications in the overall nucleic acid sequence include codon optimization of the sequence. Alterations may be made which lead to conservative substitutions within the expressed amino acid sequence. These variations can be made in complementarity determining and non-complementarity determining regions of the amino acid sequence of the fusion protein that do not affect function. Usually, additions and deletions should not be performed in the CDR3 region.

### Nucleotide sequences, vectors, AAV

Another aspect of the invention refers to a nucleotide sequence encoding the fusion protein as described herein.

Moreover, the invention refers to a vector comprising nucleotide sequence as described above.

In a specific embodiment, the vector comprises the nucleotide sequence coding for the fusion protein comprising a VP1 capsid protein, in which the cell surface marker specific VHH is integrated. The vector may further contain one or several mutations that inhibit the production of mRNA coding for VP2 and VP3.

For the production of an AAV an additional vector could be used, which is mutated, so that VP1 is not expressed, e.g. by a mutation of the VP1 start codon and which therefore codes for VP2 and VP3 but not for VP1.

The skilled person understands that this principle could be also applied for the production of AAVs in which the cell surface marker specific VHH is integrated into VP2 and VP3. Accordingly, in general, a first vector comprises the nucleotide sequence coding for a fusion protein comprising the one type of capsid protein in which the cell surface marker specific VHH is integrated, while the expression of the two other types of capsid proteins is inhibited. A second vector comprises the nucleotide sequence coding for the two other types of capsid proteins, while the expression of the capsid protein, which is modified is inhibited.

Accordingly, the invention also encompasses a kit comprising the vectors as described above.

A "vector" is any molecule or composition that has the ability to carry a nucleic acid sequence into a suitable host cell where synthesis of the encoded polypeptide can take place. Typically, and preferably, a vector is a nucleic acid that has been engineered, using recombinant DNA techniques that are known in the art, to incorporate a desired nucleic acid sequence (e.g. a nucleic acid of the invention). The vector may comprise DNA or RNA and/or comprise liposomes. The vector may be a plasmid, shuttle vector, phagemide, cosmid, expression vector, retroviral vector, lentiviral vector, adenoviral vector, AVV vector or particle and/or vector to be used in gene therapy. A vector may include nucleic acid sequences that permit it to replicate in a host cell, such as an origin of replication. A vector may also include one or more selectable marker genes and other genetic elements known to those of ordinary skill in the art. A vector preferably is an expression vector that includes a nucleic acid according to the present invention operably linked to sequences allowing for the expression of said nucleic acid.

Another aspect of the invention encompasses an AAV containing at least one of the fusion proteins comprising cell surface marker specific VHH and an adeno-associated virus (AAV) capsid protein as described herein.

### Uses

Another aspect of the invention relates to use of the fusion proteins, the nucleotide sequences, the vectors, the AAVs as described herein for improving the transduction efficiency of the AAV targeted cells.

Another aspect of the invention relates to use of the fusion proteins, the nucleotide sequences, the vectors, the AAVs as described herein for introducing a defined nucleic sequence into a target cell.

A further aspect of the invention relates to the fusion proteins, the nucleotide sequences, the vectors, the AAVs as described herein for use as a medicament.

Thus, one embodiment refers to the fusion proteins, the nucleotide sequences, the vectors, the AAVs as described herein for use in gene therapy.

In particular, one embodiment refers to the fusion proteins, the nucleotide sequences, the vectors, the AAVs as described herein for use in the treatment of cancer.

### Methods

### Flow cytometry analysis of untransfected HEK cells and cells stably transfected with CD38

HEK cells were transfected with cDNA expression vectors encoding ARTC2.2, P2X7, or CD38 and stable transfectants were selected by propagation in the presence of a suitable antibiotic and by FACS-sorting. ARTC2.2-specific, CD38-specific, and P2X7-specific VHHs (Fumey et al. 2017, Koch-Nolte et al. 2007, Danquah et al. 2016) were conjugated to Alexa647 fluorochrome using amino-link conjugation (Pierce). In cases where two distinct cell populations were mixed for analysis, one of these was labeled with the cell staining dye eFluor450 (Thermo Fisher Scientific) before mixing. Cells were incubated with fluorochrome conjugated VHHs or antibodies for 30 min at 4°C and washed before flow cytometry using a FACS Canto II (BD Biosciences). Fig. 1 shows the specific staining of transfected cells by the respective cell surface marker-specific VHHs.

### Cloning of VP1-VHH fusion proteins

The procedure for inserting a VHH into the VP1 capsid protein is illustrated schematically in Fig. 2. The VHH-coding region was amplified via PCR using a sense primer that contains the restriction site for the endonuclease *NgoMIV* and encodes a 25 AA long GS-Linker, (GGGGS)₅, and an anti-sense primer that contains the restriction site for the endonuclease *KasI* and encodes a sequence of four glycines and one alanine. The PCR fragment and 2 µg of the target vector *pRC_RR_VP1_r1c3* (provided by Junghae Suh, Rice University Houston, Texas; Judd et al. 2012) were digested with 1 µl of *NgoMIV* and *KasI* (NEB Biolabs), respectively, for 5 h at 37 °C according to the manufacturers protocol. 2 µl of the ligation samples were transformed into competent *E. coli* bacteria. Plasmid DNA was extracted using the QIAprep Spin Miniprep Kit (QIAGEN). DNA sequences were verified by sequencing. The modified *pRC_PR_VP1_r1c3* vectors containing the VHH sequences (*pRC_RR_VP1-Nb*) were mutagenized at the capsid positions R585 and R588, replacing the arginines for alanines. The mutagenesis was performed by PCR using the QuikChange II Site-Directed Mutagenesis Kit (Agilent Technologies) according to the manufacturers protocol.

### Production of AAV2, AAV2^{RA} and AAV2^{RA} containing VP1-Nb fusion proteins

The production of AAVs was performed by triple or quadruple transfection of HEK293AAV cells (Cell Biolabs) with three (AAV2, AAV2^{RA}) or four (AAV2^{RA}-Nb^{VP1}) plasmids. For the production of AAV2^{RA}, the capsid-encoding plasmid *pXX2* (provided by Martin Trepel, UKE Hamburg) was changed at the capsid positions R585 and R588 (replacing the arginines for alanines) by site directed mutagenesis using the QuikChange II Site-Directed Mutagenesis Kit (Agilent Technologies) according to the manufacturers protocol, resulting in a plasmid referred to as *pXX2_R585*/*8A.* For the production of AAV2^{RA} containing VP1-Nb fusion proteins, the same mutations were introduced into the VP2/VP3-encoding plasmid *pRC_RR_VP2-3* (provided by Junghae Suh, Houston, Texas; Judd et al. 2012) resulting in a plasmid referred to as *pRC_RR_VP2-3_R585*/*A.* For transfection, 6 × 10⁶ cells/dish were plated in 25 ml DMEM complete medium with 10 % FCS in 15 cm cell culture dishes (Cellstar, Greiner Bio-One) and cultured at 37 °C, 5 % CO₂ until 70 % confluency. The medium was replaced by 17 ml of fresh medium 1 h before transfection. Polyethyleneimine (25kD, PEI, Polysciences Inc.) was used as transfection reagent at a concentration of 0.333 mg/ml diluted in ddH₂O. The following plasmids were used for the production of AAV2 and AAV2^{RA}: 4.5 µg *pXX2* (AAV2) or 4.5 µg *pXX2_R585*/*8A* (AAV2^{RA}), 20 µg of the helper plasmid *pXX6* (provided by Martin Trepel, UKE Hamburg) and 4 µg of the transgene *scAA V-CMVeGFP* (provided by Martin Trepel, UKE Hamburg) per culture dish. For the production of AAV2^{RA} containing VP1-Nb fusion proteins, 4.5 µg *pRC_RR_VP1-Nb* and 4 µg *pRC_RR_VP2-3_R585*/*A* were used instead of *pXX2.* The corresponding plasmids were mixed in 750 µl ddH₂O before the addition of 750 µl 300 mM NaCl and mixing by vortex for 10 s. PEI was added for each transfection in fourfold molar excess to the DNA. Therefore, PEI was diluted in 750 µl ddH₂O and was also supplemented with 750 µl 300 mM NaCl and mixed for 10 s by vortex. The PEI solution was added dropwise to the DNA solution, vortexed for 10 s and incubated at room temperature for 15-20 min. The mix (3 ml) was added dropwise uniformly to the cells of one culture dish. The cells were cultured at 37 °C and 5 % CO₂. After 24 h, the medium was replaced with 25 ml of fresh medium. After three days, AAVs were harvested from the cell supernatant and cell lysates.

### Recovery of recombinant AAVs from cell culture supernatants and cell lysates

Transfected HEK293AAV cells were carefully detached from the culture dish with a cell scraper three days after medium exchange and were transferred with the culture supernatant into a 50 ml falcon tube and centrifuged for 5 minutes at 1200 rpm, 4° C. The supernatant was transferred to a fresh 50 ml falcon tube and the cell pellet was stored on ice until the cells were lysed. For the precipitation of AAVs from the supernatant, 10 g PEG-8000 and 5.8 g NaCl were added per 100 ml supernatant (about 10 % w/v PEG-8000 and 1 M NaCl) and incubated rolling overnight at 4 °C. The next day, the suspensions were centrifuged at 4,600 rpm for 30 min. The supernatant was discarded, and the pellet-containing tubes were again centrifuged for 3 min at 4,600 rpm in order to completely remove remaining supernatant. The AAV-containing pellet was resuspended in 1 ml PBS-MK (PBS with 1 mM MgCl₂ and 2.5 mM KCl₂). For the recovery of AAVs from cell lysates, cell pellets were resuspended in 1 ml PBS-MK and transferred to 15 ml falcon tubes. Cells were lysed by three cycles of freeze/thaw (5-10 min freezing in dry ice surrounded by 99% ethanol, 5-10 minutes thawing in a 37 °C water bath). Subsequently, DNA and RNA in supernatants and lysates were digested using the endonuclease benzonase (Sigma, E1014, 250 U/µl) for 30 min at 37 °C (50 U/ml supernatant or lysate). Remaining insoluble material was eliminated by centrifugation for 15 min at 4,600 rpm. Viral genome titers were determined by quantitative real-time PCR. Lysates and supernatants were stored at 80 °C._For detecting Nb-VP1 fusion proteins incorporated into the AAV2^{RA} capsids, AAVs were immunoprecipitated from transfected cell supernatants using the AAV-specific antibody scFvA20-rbFc (the scFv of mAb A20 (McCraw et al. 2012) fused to the hinge, CH3 and CH3 domains of a rabbit IgG antibody) immobilized to protein G sepharose beads. Eluted proteins were size-fractionated by SDS-PAGE and transferred to a PVDF membrane. The detection of capsid proteins was carried out with the mAb B1 (Wistuba et al. 1995) (provided as hybridoma supernatant by Martin Trepel), that recognizes an epitope at the C-terminus of all three capsid proteins (Fig. 3A). The VP1-Nb fusion proteins show the expected shift in Mr of ∼15 kDa compared to the parental VP1 (100 kDa vss 85 kDa). For the detection of AAV2^{RA}-1c81^{VP1} bound to HEK cells, untransfected HEK293 cells were mixed in a ratio of 10:1 with eFluor450-labeled P2X7-expressing HEK293 cells and incubated with AAV-containing cell culture supernatants (1 × 10⁹ vg) for 30 min on ice. Unbound AAVs were removed by washing the cells twice with PBS containing 0.02 % BSA. Bound virions were detected by incubation with an AlexaFluor647-conjugated AAV-specific antibody (scFvA20-rbFc) (20 min at 4 °C). Fluorescence was analyzed by flow cytometry using the FACS Canto II and FlowJo software (BD Biosciences) (Fig. 3B). The results confirm the specific binding of AAV2^{RA}-1c81^{VP1} to P2X7-transfected HEK cells but not to untransfected HEK cells.

### Transduction of HEK cells with AAV2^{RA}-Nb^{VP1}

To evaluate the transduction of AAV2^{RA} containing VP1 fusion proteins with inserted CD38- or ARTC2.2-specific VHHs 370 and s-14, respectively, HEK293 cells stably transfected with ARTC2.2 or CD38 were mixed 1:1 and seeded in 24-well cell culture plates (1x10⁵ cells/well) in DMEM complete medium with 10 % FCS. After 2 h, AAVs in cell culture supernatants were diluted in medium and added to the cells at a final volume of 500 µl (500 vg/cell). 48 h later the expression of the transgene GFP was analyzed by flow cytometry using the FACS Canto II and FlowJo software (BD Biosciences) (Fig. 4A). To distinguish the two cell populations, cells were counterstained 30 min before analysis with AlexaFluor647-conjugated CD38-specific antibody JK36-rbFc. The results show specific transduction of CD38-transfected HEK cells with AAV2^{RA}-370^{VP1} and of ARTC2.2-transfected HEK cells with AAV2^{RA}-s-14^{VP1}. In order to assess the transduction of AAV2^{RA} containing VP1 fusion proteins with the inserted P2X7-specific VHH 1c81, untransfected HEK293 cells and HEK cells stably transfected with P2X7 were seeded in 24-well cell culture plates (1 x 10⁵ cells/well) in DMEM complete medium with 10 % FCS and incubated to about 80 % confluency at 37 °C, 5 % CO₂. AAVs in cell culture supernatants were diluted in medium and added to the cells at a final volume of 500 µl (1,000 vg/cell). 48 h later the expression of the transgene GFP was analyzed by flow cytometry using the FACS Canto II and FlowJo software (BD Biosciences) (Fig. 4B). The results show specific transduction of P2X7-transfected HEK cells with AAV2^{RA}-1c81^{VP1}.

### Generation of Llama IgH transgenic mice

Two overlapping clones (V03 and F07) carrying variable and/or constant segments of the immunoglobulin heavy chain gene locus were isolated from a *Lama glama* genomic DNA-library in the BAC vector pCC1 (Epicentre) and sequenced. Regions HS3b and HS4 of the mouse locus control region (LCR, alpha-enhancer) were inserted into the 3' end of V03. For this, HS3b and HS4 were amplified from a plasmid encoding the essential parts of the murine LCR (provided by Michel Cogné, Limoges, France, Pinaud et al. 2001) using PCR primers with 50 bp overhangs homologous to the BAC insertion site. The insertion of the amplified construct into V03 was carried out using the Red®/ET recombineering technology (GeneBridges). The *Lama glama* Cδ pseudogene and the associated switch region was replaced by a spectinomycin resistance cassette. The selection cassette was PCR amplified with 50 bp overhangs homologous to the sequences flanking the *Lama glama* Cδ region. Exchange of the Cδ region by the amplified selection cassette was carried out using the Red®/ET recombineering techology. The CH1 domain of the *Lama glama* Cµ locus was replaced by an ampicilin resistance cassette flanked by the two loxP mutants lox71 and lox66 (Parrish et al. 2011). This construct was synthesized with 50 bp overhangs homologous to sequences flanking the Cµ CH1 domain. Recombination was carried out using the recombineering strain *E.coli SW106* (provided by Neal Copeland, Frederick, MD, USA, Warming et al. 2005). The homologous recombination was followed by a Cre/loxP recombination to delete the ampicillin selection cassette from V03. The spectinomycin selection cassette at the position of the Cδ pseudogene was replaced by the *Lama glama* Cy2b isotype obtained from F07. For this, Cy2b was cloned into the vector *pBluescript II KS (*+*)*/*LIC.* To equip the Cy2b locus with homology arms, a gene synthesis construct with two homology regions each flanked by *pBluescript II KS (*+*)*/*LIC* compatible cloning sites (*ClaI* and *BstZ17I*) was designed. The 270 bp 3'-homology region was genetically fused to an ampicillin selection cassette flanked by the two loxP mutants lox71 and lox66. The construct was synthesized and cloned into the vector pUC57. The 285 bp 5 'homology region was isolated by *ClaI* restriction digestion and cloned into the ClaI site upstream of the Cy2b locus in *pBluescript II KS (*+*)*/*LIC.* The resulting plasmid was used as the target vector for the insertion of the 270 bp 3 'homology arm fused to the ampicillin selection cassette. This construct was isolated by *BstZ17I* restriction digestion and cloned into the *BstZ17I* site downstream of the Cy2b locus in *pBluescript II KS (*+*)*/*LIC.* A 5' homology arm of 285 bp size containing a *NruI* restriction site was synthezised as complementary primer sequences. Hybridization of the complementary single stranded DNA molecules led to a restriction site-specific overhang allowing site-specific insertion of the homology arm upstream of the Cy2b locus. An ampicilin resistance cassette flanked by the two loxP mutants lox71 and lox66 fused to a 270 bp 3' homology arm containing a second *NruI* restriction site was synthesized as double stranded DNA and inserted downstream of the Cy2b locus. The entire construct with both homology arms and the floxed ampicillin selection cassette was isolated using the blunt end *NruI* restriction sites encoded in the homology arms. Recombination was carried out using the recombineering strain *E.coli SW106.* The homologous recombination was followed by a Cre/loxP recombination to delete the ampicillin selection cassette downstream of the Cy2b locus. The modified BAC was purified using the PhasePrep BAC DNA Kit (Sigma-Aldrich) followed by a phenol/chloroform extraction and linearization with *NotI.* The linear construct was injected into fertilized oocytes of C57BL/6J x CBA mice with pronuclear injection (Nagy et al. 2003). Founder mice were backcrossed to Ig-deficient J_{H}T-mice (provided by Klaus Rajewsky, Gu et al. 1993). Transgenic mice were identified by PCR from ear clip DNA using Platinum blue PCR super mix (Invitrogen). Pairs of PCR primers specific for the 5' and 3' transgene ends were used to verify germline transmission under the following conditions: 30x (95°C 20 secs, 60°C 30 secs, 72°C 60 secs), 72°C 10 mins. The knockout of the endogenous IgH locus (deletion of the J-elements and the µ-enhancer) of Ig-deficent J_{H}T-mice was verified using the same PCR conditions. Primer pairs specific for the mutated region of the IgH locus are described in the technical support protocol of Stock No: 002438 (The Jackson Laboratory).

### Immunization of Lama-IgH transgenic mice and cloning of AAV1-specific VHH

Lama-IgH transgenic mice received five immunizations with AAV1 (10¹¹ vg/200 µl PBS i.m.) in three week intervals. Three days after the final boost, mice were sacrificed and cells were isolated from spleen, lymph nodes and bone marrow. RNA was purified using the innuPREP RNA Mini Kit (analytik Jena) and transcribed into cDNA using reverse transcriptase (Gibco) and random hexamer primers (GE Healthcare). The VHH repertoire was PCR amplified from cDNA (50 ng/reaction) using IgM-specific and IgG-specific primers (39 cycles: 95°C 30 s, 57°C 20 s, 70° 20 s) and cloned into the pCSE2.5 expression vector (Schirrmann et al. 2010) upstream of a cassette encoding the hinge, CH2, and CH3 domains of rabbit IgG. Individual clones were sequenced and transiently transfected into HEK-6E cells (Zhang et al. 2009). Cell supernatants were harvested 6 days after transfection. Proteins in cell supernatants were analyzed by SDS-PAGE and Coomassie staining (Fig. 5A). The results reveal efficient production of heavy chain antibodies. Cell supernatants were analyzed for antigen-specific heavy chain antibodies by ELISA. Wells were coated with AAV1 or AAV2 (10⁹ vg/100 µl PBS) or an irrelevant control protein (ARTC2.2 or BSA, 100 ng/100 µl PBS). Wells were blocked with albumin and were then incubated with individual cell supernatants (diluted 1:20 in PBS). An ARTC2.2-specific VHH-rabbit IgG heavy chain antibody (s+16a, Koch-Nolte et al. 2007) and an AAV2-specific scFv-rabbit IgG antibody (scFv of mAb A20, McCraw et al. 2012) were used as controls. Wells were washed and bound VHH-rabbit IgG heavy chain antibodies were detected with peroxidase-conjugated secondary antibodies (donkey anti-rabbit IgG, GE Healthcare) and TMB as substrate. The results show specific binding of VHHs 147, 174, 176, and 53 to AAV1; and of VHHs 11, 90 and 152 to AAV1 and AAV2.

### Cloning and production of recombinant adapter proteins

Cloning into the mammalian pCSE2.5 expression vector (Schirrmann et al. 2010) was performed by three-way-ligation of an N-terminal membrane protein-specific VHH and a C-terminal AAV-specific scFv or VHH. For the scFv, the amino acid sequence of the VH and VL domains of the monoclonal murine antibody A20 (McCraw et al. 2012) was transcribed to a nucleotide sequence using the GeneOptimizer™ Software containing a 15 AA long GS peptide linker, (GGGGS)₃, between the VH and VL domain. The DNA fragment was chemically synthesized by the company Thermo Fisher Scientific and delivered in the *pMA-T* vector. In order to clone the fusion proteins, the N-terminal VHH coding region was amplified by PCR from the vector *pCSE2.5* using a sense primer, that contains the recognition site of the endonuclease *PciI*, and an antisense primer, that encodes a 10 AA GS linker peptide, (GGGGS)₂, including the restriction site of the endonuclease *BamHI.* The amplification of the scFv of mAb A20 was carried out using a sense primer also containing the restriction site of *BamHI* followed by the sequence for a 10 AA GS linker peptide and using a standard sequencing primer of the vector *pCSE2.5* as anti-sense primer. The amplicons were each isolated with the kit Nucleospin Extract II (Macherey-Nagel) according to the manufacturer. The VHH-encoding amplicon was cleaved with the enzymes *PciI* and *BamHI* (NEB), while the scFv-encoding amplicon was digested with *BamHI* and *NotI* (NEB) according to the manufacturers protocol. The target vector *pCSE2.5_His-c-myc* was enzymatically cleaved with the endonucleases *NcoI* and *NotI* and isolated by agarose gel electrophoresis. The endonucleases *NcoI* and *PciI* generate complementary nucleotide overhangs of the DNA. The ligation was performed for 16 h at 14 °C with both amplicons in a molar ratio of 1.5:1.5:1 to the target vector using the T4 ligase. 2 µl of the ligation samples were transformed into competent *E.coli* bacteria. Plasmid DNA was extracted using the QIAprep Spin Miniprep Kit (QIAGEN). DNA sequences were verified by sequencing. To clone the fusion proteins consisting of two VHHs, the scFv-coding sequence was removed from the vectors by restriction digestion with the enzymes *BamHI* and *NotI* and the VHH-encoding vectors were used as backbones. The AAV-specific VHH-coding regions were amplified by PCR using a sense primer, that contains the restriction site for *BamHI* followed by a sequence encoding a 10 AA GS linker peptide, (GGGGS)₂, and a sequence encoding for four glycine and one asparagine containing the restriction site for the enzyme *PciI* to allow a subsequent easy exchange of the AAV-specific VHH. The amplicon was ligated with the *pCSE2.5* vector backbone in a ratio of 3:1 using the T4 ligase for 16 h at 14 °C. 2 µl of each ligation sample was transformed into competent *E. coli* bacteria. Plasmid DNA was extracted using the QIAprep Spin Miniprep Kit (QIAGEN). DNA sequences were verified by sequencing. Individual clones were transiently transfected into HEK-6E cells (Zhang et al. 2009). Cell supernatants were harvested 6 days after transfection. Proteins in cell supernatants were analyzed by SDS-PAGE and Coomassie staining (Fig. 6A). Adapter proteins were purified from cell supernatants using immobilized metal affinity chromatography and purity was verified by SDS-PAGE and Coomassie staining (Fig. 6B).

### Transduction of HEK cells with AAV and adapter proteins

Untransfected HEK293 cells or HEK293 cells stably transfected with P2X7 or a mixture of ARTC2.2-transfected HEK cells and eFluor450-labeled CD38-transfected HEK cells were seeded in 96-well (2 x 10⁴ cells/well) cell culture dishes. Subsequently, cells were incubated with GFP-encoding AAV1 (10,000 vg/cell) (Fig. 7) or AAV2^{RA} (300 vg/cell) (Fig. 8) preincubated with titrated HEK293-6E cell supernatants containing adapter proteins (30 min, 4 °C). 48 h later, the expression of GFP was analyzed by flow cytometry using the FACS Canto II and FlowJo software (BD Biosciences). The results show that adapters containing the AAV1/AAV2 dual specific VHH 152 specifically enhance transduction of target-transfected HEK cells by both, AAV1 (Fig. 7) and AAV2^{RA} (Fig. 8). Adapters containing the AAV1-specific VHH 174 specifically enhance transduction of target-transfected HEK cells by AAV1 (Fig. 7) but not by AAV2^{RA} (Fig. 8). Adapters containing the AAV2-specific scFv A20 (scFv of mAb A20, McCraw et al. 2012) and the CD38-specific VHH 370 (SEQ ID NO:10) slightly enhance the transduction of CD38 expressing HEK cells by AAV2^{RA} (Fig. 8B).

The description further contains the following embodiments:
Embodiment 1. Fusion protein comprising a cell surface marker specific VHH.
Embodiment 2. Fusion protein according to embodiment 1 further comprising an adeno-associated virus (AAV) specific VHH.
Embodiment 3. The fusion protein according to any one of the preceding embodiments, wherein the serotype of the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof.
Embodiment 4. The fusion protein according to any one of the preceding embodiments, wherein the serotype of the AAV is AAV1 and/or AAV2 or variants thereof.
Embodiment 5. The fusion protein according to embodiment 4, wherein the serotype of the AAV is AAV1.
Embodiment 6. The fusion protein according to embodiment 5, wherein the sequence of the adeno-associated virus (AAV) specific VHH is selected from the group consisting of SEQ ID NO: 20 to 24 or sequences having at least 80% identity thereto.
Embodiment 7. The fusion protein according to embodiment 4, wherein the serotype of the AAV is AAV1 or AAV2.
Embodiment 8. The fusion protein according to embodiment 7, wherein the sequence of the adeno-associated virus (AAV) specific VHH is selected from the group consisting of SEQ ID NO: 25 to 27 or sequences having at least 80% identity thereto.
Embodiment 9. The fusion protein according to any one of the preceding embodiments, wherein the cell surface marker is a membrane protein.
Embodiment 10. The fusion protein according to any one of the preceding embodiments, wherein the membrane protein is selected from the group consisting of multi-span homo trimer membrane protein, type-II single span membrane protein, GPI-anchor membrane protein.
Embodiment 11. The fusion protein according to embodiment 10, wherein the multi-span trimer membrane protein is P2X7.
Embodiment 12. The fusion protein according to embodiment 10, wherein the type-II single span membrane protein is CD38.
Embodiment 13. The fusion protein according to embodiment 10, wherein the GPI-anchor membrane protein is ARTC2.2.
Embodiment 14. The fusion protein according to embodiment 11, wherein the membrane protein is P2X7 and the AAV is of the AAV1 serotype.
Embodiment 15. The fusion protein according to embodiment 14, wherein the protein sequence of the fusion protein is set out in SEQ ID NO:4 or is at least 80% identical thereto.
Embodiment 16. The fusion protein according to embodiment 11, wherein the membrane protein is P2X7 and the AAV is of the AAV1 or AAV2 serotype.
Embodiment 17. The fusion protein according to embodiment 16, wherein the protein sequence of the fusion protein is set out in SEQ ID NO:5 or is at least 80% identical thereto.
Embodiment 18. The fusion protein according to embodiment 12, wherein the membrane protein is CD38 and the AAV is of the AAV1 serotype.
Embodiment 19. The fusion protein according to embodiment 18, wherein the protein sequence of the fusion protein is set out in SEQ ID NO:6 or is at least 80% identical thereto.
Embodiment 20. The fusion protein according to embodiment 12, wherein the membrane protein is CD38 and the AAV is of the AAV1 or the AAV2 serotype.
Embodiment 21. The fusion protein according to embodiment 20, wherein the protein sequence of the fusion protein is set out in SEQ ID NO:7 or is at least 80% identical thereto.
Embodiment 22. The fusion protein according to embodiment 13, wherein the membrane protein is ARTC2.2 and the AAV is of the AAV1 serotype.
Embodiment 23. The fusion protein according to embodiment 22, wherein the protein sequence of the fusion protein is set out in SEQ ID NO:8 or is at least 80% identical thereto.
Embodiment 24. The fusion protein according to embodiment 13, wherein the membrane protein is ARTC2.2 and the AAV is of the AAV1 or AAV2 serotype.
Embodiment 25. The fusion protein according to embodiment 24, wherein the protein sequence of the fusion protein is set out in SEQ ID NO:9 or is at least 80% identical thereto.
Embodiment 26. The fusion protein according to any one of the preceding embodiments, wherein the fusion protein comprises a linker between the cell surface marker specific VHH and the VHH specific for AAV.
Embodiment 27. The fusion protein according to any one of the preceding embodiments, wherein the linker is a peptide linker.
Embodiment 28. The fusion protein according to any one of the preceding embodiments, wherein the sequence of the peptide linker is set out in SEQ ID NO: 19.
Embodiment 29. Fusion protein according to embodiment 1, wherein the fusion protein comprises a cell surface marker specific VHH and an adeno-associated virus (AAV) capsid protein.
Embodiment 30. The fusion protein according to embodiment 29, wherein the serotype of the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof.
Embodiment 31. The fusion protein according to any one of embodiments 29 to 30, wherein the serotype of the AAV is AAV1 and/or AAV2 or variants thereof.
Embodiment 32. The fusion protein according to any one of embodiments 29 to 31, wherein the AAV capsid protein is VP1 or VP2.
Embodiment 33. The fusion protein according to embodiment 32, wherein the AAV capsid protein is VP1.
Embodiment 34. The fusion protein according to any one of embodiments 29 to 33, wherein the cell surface marker is a membrane protein.
Embodiment 35. The fusion protein according to embodiment 34, wherein the membrane protein is selected from the group consisting of multi-span homo trimer membrane protein, type-II single span membrane protein, GPI-anchor membrane protein.
Embodiment 36. The fusion protein according to embodiment 35, wherein the multi-span trimer membrane protein is P2X7.
Embodiment 37. The fusion protein according to embodiment 35, wherein the type-II single span membrane protein is CD38.
Embodiment 38. The fusion protein according to embodiment 35, wherein the GPI-anchor membrane protein is ARTC2.2.
Embodiment 39. The fusion protein according to embodiment 36, wherein the membrane protein is P2X7 and the AAV is of the AAV2 serotype.
Embodiment 40. The fusion protein according to embodiment 39, wherein the protein sequence of the fusion protein is set out in SEQ ID NO:1 or is at least 80% identical thereto.
Embodiment 41. The fusion protein according to embodiment 37, wherein the membrane protein is CD38 and the AAV is of the AAV2 serotype.
Embodiment 42. The fusion protein according to embodiment 41, wherein the protein sequence of the fusion protein is set out in SEQ ID NO:2 or is at least 80% identical thereto.
Embodiment 43. The fusion protein of embodiment 40, wherein the membrane protein is ARTC2.2 and the AAV is of the AAV2 serotype.
Embodiment 44. The fusion protein according to embodiment 42, wherein the protein sequence of the fusion protein is set out in SEQ ID NO:3 or is at least 80% identical thereto.
Embodiment 45. Nucleotide sequence encoding the fusion protein according to any one of embodiments 1 to 44.
Embodiment 46. Vector comprising nucleotide sequence according to embodiment 45.
Embodiment 47. AAV containing at least one of the fusion proteins according to embodiments 29 to 44.
Embodiment 48. Use of the fusion proteins according to any one of the embodiments 1 to 44, the nucleotide sequence according to embodiment 45 or the vector according to embodiment 46, the AAV according to embodiment 47 for improving the transduction efficiency of the AAV targeted cells.
Embodiment 49. Use of the fusion proteins according to any one of the embodiments 1 to 44, the nucleotide sequence according to embodiment 45 or the vector according to embodiment 46, the AAV according to embodiment 47 for introducing a defined nucleic sequence into a target cell.

### REFERENCES

Bartlett et al. (1999) Targeted adeno-associated virus vector transduction of nonpermisive cells mediated by a bispecific F(ab'gamma)2 antibody. Nat Biotechnol, 17:181-6.
Boucas et al. (2009) Engineering andeno-associated virus serotype 2-based targeting vectors using a new insertion site position 453- and single point mutations. J Gene Med 11:1103-13
Danquah et al. (2016) Nanobodies that block gating of the P2X7 ion channel ameliorate inflammation. Sci Transl Med, 8:366ra162.
Els-Conrath et al. (2001) Camel singledomain antibodies as modular building units in bispecific and bivalent antibody constructs. J Biol Chem, 276: 7346-50.
Fumey et al. (2017) Nanobodies effectively modulate the enzymatic activity of CD38 and allow specific imaging of CD38+ tumors in mouse models in vivo. Sci Rep, 7:14289.
Girod et al. (2002) The VP1 capsid protein of adeno-associated virus type 2 is carrying a phospholipase A2 domain required for virus infectivity. J Gen Virol. 83(Pt5):973-8.
Gu et al. (2003) Independent control of immunoglobulin switch recombination at individual switch regions evidenced through Cre-loxP-mediated gene targeting. Cell 73(6):1155-1164 (1993).
Judd et al. (2012) Random Insertion of mCherry Into VP3 Domain of Adeno-associated Virus Yields Fluorescent Capsids With no Loss of Infectivity. Mol Ther Nucleic Acids 1: e54.
Koch-Nolte et al. (2007) Single domain antibodies from llama effectively and specifically block T cell ecto-ADP-ribosyltransferase ART2.2 in vivo. FASEB J, 21:3490-8.
McCraw et al. (2012) Structure of adeno-associated virus-2 in complex with neutralizing monoclonal antibody A20. Virology 431(1-2):40-9.
Münch et al. (2013) Displaying high-affinity ligands on adeno-associated viral vectors enables tumor cell-specific and safe gene transfer. Mol Ther 21:109-18
Nagy et al. (2003). Manipulating the Mouse Embryo: A Laboratory Manual. Cold Spring Harbor Laboratory Press, New York.
Parrish et al. (2011) BAC modification through serial or simultaneous use of CRE/Lox technology. J Miomed Biotechnol 2011:924068
Pinaud et al., (2001) Localization of the 3' IgH locus elements that effect long-distance regulation of class switch recombination. Immunity 15:187-199
Schirrmann et al. (2010) Transient Production of scFv-Fc Fusion Proteins in Mammalian Cells. in Antibody Engineering Vol. 2. (eds. R. Kontermann & S. Dübel) 387-398; Springer-Verlag, Berlin Heidelberg
Warming et al. (2005) Simple and highly efficient BAC recombineering using galK selection. Nucleic Acids Res 24: 33(4):e36)
Warrington et al. (2004) Adeno-associated virus type 2 VP2 capsid protein is nonessential and can tolerate large peptide insertions at its N terminus. J Virol. 78(12):6595-609.
Wistuba et al. (1995) Intermediates of adeno-associated virus type 2 assembly: identification of soluble complexes containing Rep and Cap proteins. J Virol. 1995 Sep;69(9):5311-9.
Zhang et al. (2009) Production of chimeric heavy-chain antibodies. Methods Mol Biol 525, 323-336

## Claims

1. Fusion protein comprising cell surface marker specific variable heavy chain domain of an heavy chain antibody (VHH).

2. Fusion protein according to claim 1 further comprising an adeno-associated virus (AAV) specific VHH.

3. The fusion protein according to any one of the preceding claims, wherein the cell surface marker is a membrane protein.

4. The fusion protein according to claim 3, wherein the membrane protein is P2X7 and the AAV is of the AAV1 serotype, wherein preferably the protein sequence of the fusion protein is set out in SEQ ID NO:4 or is at least 80% identical thereto.

5. The fusion protein according to claim 3, wherein the membrane protein is P2X7 and the AAV is of the AAV1 or AAV2 serotype, wherein preferably the protein sequence of the fusion protein is set out in SEQ ID NO:5 or is at least 80% identical thereto.

6. Fusion protein according to claim 1, wherein the fusion protein comprises a cell surface marker specific VHH and an adeno-associated virus (AAV) capsid protein.

7. The fusion protein according to claim 6, wherein the AAV capsid protein is VP1.

8. The fusion protein according to claim 6 or 7, wherein the cell surface marker is a membrane protein.

9. The fusion protein according to claim 8, wherein the membrane protein is P2X7 and the AAV is of the AAV2 serotype, wherein preferably the protein sequence of the fusion protein is set out in SEQ ID NO:1 or is at least 80% identical thereto.

10. The fusion protein according to claim 8, wherein the membrane protein is CD38 and the AAV is of the AAV2 serotype wherein preferably the protein sequence of the fusion protein is set out in SEQ ID NO:2 or is at least 80% identical thereto.

11. Nucleotide sequence encoding the fusion protein according to any one of claims 1 to 10.

12. Vector comprising nucleotide sequence according to claim 11.

13. AAV containing at least one of the fusion proteins according to claims 6 to 10.

14. Use of the fusion proteins according to claims 1 to 10, the nucleotide sequence according to claim 11 or the vector according to claim 12, the AAV according to claim 13 for improving the transduction efficiency of the AAV targeted cells.

15. Use of the fusion proteins according to claims 1 to 10, the nucleotide sequence according to claim 11 or the vector according to claim 12, the AAV according to claim 13 for introducing a defined nucleic sequence into a target cell.


SEQUENCE LISTING

<110>  Universitätsklinikum Hamburg-Eppendorf

<120>  Fusion proteins comprising a cell surface marker specific VHH

<130>  T08231EP / RN

<140>
<141>

<160>  31

<170>  PatentIn version 3.5

<210>  1
<211>  889
<212>  PRT
<213>  Artificial Sequence

<220>
<223>  VP1-nanobody fusion protein

<400>  1

Met Ala Ala Asp Gly Tyr Leu Pro Tyr Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Pro Val Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Leu Gly Gln Pro Pro
            180                 185                 190

Ala Ala Pro Ser Gly Leu Gly Thr Asn Thr Met Ala Thr Gly Ser Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Arg Leu Asn Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Thr Phe Ser Tyr Thr Phe Glu

                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Ser Arg Thr
        435                 440                 445

Asn Thr Pro Ser Ala Gly Gly Gly Gly Ser Gly Gly Gly Gly Ser Gly
    450                 455                 460

Gly Gly Gly Ser Gly Gly Gly Gly Ser Gly Gly Gly Gly Ser Glu Val
465                 470                 475                 480

Gln Leu Val Glu Ser Gly Gly Lys Leu Val Gln Ala Gly Gly Ser Leu
                485                 490                 495

Arg Leu Ser Cys Ser Ala Ser Gly Arg Thr Phe Ser Phe Ser Thr Ser
            500                 505                 510

Thr Met Gly Trp Phe Arg Gln Ala Pro Gly Lys Glu Leu Glu Phe Val
        515                 520                 525

Ala Ala Ile Asp Trp Ser Asp Phe Asn Thr Tyr Tyr Ala Asp Ser Val
    530                 535                 540

Lys Gly Arg Phe Thr Ile Ser Arg His Asn Pro Arg Asn Ser Val Tyr
545                 550                 555                 560

Leu Gln Leu Asn Ser Leu Lys Pro Glu Asp Thr Ala Val Tyr Tyr Cys
                565                 570                 575

Ala Ala His Ser Glu Thr Arg Gly Gly Thr Arg Tyr Phe Asp Arg Pro
            580                 585                 590

Ser Leu Tyr Asn Tyr Trp Gly Gln Gly Thr Gln Val Thr Val Ser Ser
        595                 600                 605

Gly Gly Gly Gly Ala Leu Gln Phe Ser Gln Ala Gly Ala Ser Asp Ile
    610                 615                 620

Arg Asp Gln Ser Arg Asn Trp Leu Pro Gly Pro Cys Tyr Arg Gln Gln
625                 630                 635                 640

Arg Val Ser Lys Thr Ser Ala Asp Asn Asn Asn Ser Glu Tyr Ser Trp
                645                 650                 655

Thr Gly Ala Thr Lys Tyr His Leu Asn Gly Arg Asp Ser Leu Val Asn
            660                 665                 670

Pro Gly Pro Ala Met Ala Ser His Lys Asp Asp Glu Glu Lys Phe Phe
        675                 680                 685

Pro Gln Ser Gly Val Leu Ile Phe Gly Lys Gln Gly Ser Glu Lys Thr
    690                 695                 700

Asn Val Asp Ile Glu Lys Val Met Ile Thr Asp Glu Glu Glu Ile Arg
705                 710                 715                 720

Thr Thr Asn Pro Val Ala Thr Glu Gln Tyr Gly Ser Val Ser Thr Asn
                725                 730                 735

Leu Gln Ala Gly Asn Ala Gln Ala Ala Thr Ala Asp Val Asn Thr Gln
            740                 745                 750

Gly Val Leu Pro Gly Met Val Trp Gln Asp Arg Asp Val Tyr Leu Gln
        755                 760                 765

Gly Pro Ile Trp Ala Lys Ile Pro His Thr Asp Gly His Phe His Pro
    770                 775                 780

Ser Pro Leu Met Gly Gly Phe Gly Leu Lys His Pro Pro Pro Gln Ile
785                 790                 795                 800

Leu Ile Lys Asn Thr Pro Val Pro Ala Asn Pro Ser Thr Thr Phe Ser
                805                 810                 815

Ala Ala Lys Phe Ala Ser Phe Ile Thr Gln Tyr Ser Thr Gly Gln Val
            820                 825                 830

Ser Val Glu Ile Glu Trp Glu Leu Gln Lys Glu Asn Ser Lys Arg Trp
        835                 840                 845

Asn Pro Glu Ile Gln Tyr Thr Ser Asn Tyr Asn Lys Ser Val Asn Val
    850                 855                 860

Asp Phe Thr Val Asp Thr Asn Gly Val Tyr Ser Glu Pro Arg Pro Ile
865                 870                 875                 880

Gly Thr Arg Tyr Leu Thr Arg Asn Leu
                885

<210> 2
<211> 875
<212> PRT

<213>  Artificial Sequence

<220>
<223>  VP1-nanobody fusion protein

<400>  2

Met Ala Ala Asp Gly Tyr Leu Pro Tyr Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Pro Val Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Leu Gly Gln Pro Pro
            180                 185                 190

Ala Ala Pro Ser Gly Leu Gly Thr Asn Thr Met Ala Thr Gly Ser Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Arg Leu Asn Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Thr Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Ser Arg Thr
        435                 440                 445

Asn Thr Pro Ser Ala Gly Gly Gly Gly Ser Gly Gly Gly Gly Ser Gly
    450                 455                 460

Gly Gly Gly Ser Gly Gly Gly Gly Ser Gly Gly Gly Gly Ser Asp Val
465                 470                 475                 480

Gln Leu Gln Ala Ser Gly Gly Gly Ser Val Gln Ala Gly Gly Ser Leu
                485                 490                 495

Thr Leu Ser Cys Arg Tyr Ser Gly Gly Phe Val Phe Arg Val Ser Ser
            500                 505                 510

Met Gly Trp Tyr Arg Gln Ala Pro Gly Asn Gln Arg Glu Leu Ile Ala
        515                 520                 525

Thr Ile Thr Val Gly Gly Lys Thr Asn Tyr Lys Asp Ser Val Gln Gly
    530                 535                 540

Arg Phe Ile Ile Ser Arg Asn Asp Thr Thr Val Thr Leu Gln Met Asn
545                 550                 555                 560

Arg Leu Gln Pro Glu Asp Thr Ala Val Tyr Tyr Cys Asn Leu Ala Ser
                565                 570                 575

Thr Ala Val Gly Ala Asp Thr Trp Gly Gln Gly Thr Arg Val Thr Val
            580                 585                 590

Ser Ser Gly Gly Gly Gly Ala Leu Gln Phe Ser Gln Ala Gly Ala Ser
        595                 600                 605

Asp Ile Arg Asp Gln Ser Arg Asn Trp Leu Pro Gly Pro Cys Tyr Arg
    610                 615                 620

Gln Gln Arg Val Ser Lys Thr Ser Ala Asp Asn Asn Asn Ser Glu Tyr
625                 630                 635                 640

Ser Trp Thr Gly Ala Thr Lys Tyr His Leu Asn Gly Arg Asp Ser Leu
                645                 650                 655

Val Asn Pro Gly Pro Ala Met Ala Ser His Lys Asp Asp Glu Glu Lys
            660                 665                 670

Phe Phe Pro Gln Ser Gly Val Leu Ile Phe Gly Lys Gln Gly Ser Glu
        675                 680                 685

Lys Thr Asn Val Asp Ile Glu Lys Val Met Ile Thr Asp Glu Glu Glu
    690                 695                 700

Ile Arg Thr Thr Asn Pro Val Ala Thr Glu Gln Tyr Gly Ser Val Ser
705                 710                 715                 720

Thr Asn Leu Gln Ala Gly Asn Ala Gln Ala Ala Thr Ala Asp Val Asn
                725                 730                 735

Thr Gln Gly Val Leu Pro Gly Met Val Trp Gln Asp Arg Asp Val Tyr
            740                 745                 750

Leu Gln Gly Pro Ile Trp Ala Lys Ile Pro His Thr Asp Gly His Phe
        755                 760                 765

His Pro Ser Pro Leu Met Gly Gly Phe Gly Leu Lys His Pro Pro Pro
    770                 775                 780

Gln Ile Leu Ile Lys Asn Thr Pro Val Pro Ala Asn Pro Ser Thr Thr
785                 790                 795                 800

Phe Ser Ala Ala Lys Phe Ala Ser Phe Ile Thr Gln Tyr Ser Thr Gly
                805                 810                 815

Gln Val Ser Val Glu Ile Glu Trp Glu Leu Gln Lys Glu Asn Ser Lys
            820                 825                 830

Arg Trp Asn Pro Glu Ile Gln Tyr Thr Ser Asn Tyr Asn Lys Ser Val
        835                 840                 845

Asn Val Asp Phe Thr Val Asp Thr Asn Gly Val Tyr Ser Glu Pro Arg
    850                 855                 860

Pro Ile Gly Thr Arg Tyr Leu Thr Arg Asn Leu
865                 870                 875

<210>  3
<211>  881
<212>  PRT
<213>  Artificial Sequence

<220>
<223>  VP1-nanobody fusion protein

<400>  3

Met Ala Ala Asp Gly Tyr Leu Pro Tyr Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Pro Val Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Leu Gly Gln Pro Pro
            180                 185                 190

Ala Ala Pro Ser Gly Leu Gly Thr Asn Thr Met Ala Thr Gly Ser Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Arg Leu Asn Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Thr Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Ser Arg Thr
        435                 440                 445

Asn Thr Pro Ser Ala Gly Gly Gly Gly Ser Gly Gly Gly Gly Ser Gly
    450                 455                 460

Gly Gly Gly Ser Gly Gly Gly Gly Ser Gly Gly Gly Gly Ser Gln Val
465                 470                 475                 480

Gln Leu Gln Gln Ser Gly Gly Gly Leu Val Gln Ala Gly Gly Ser Leu
                485                 490                 495

Thr Leu Ser Cys Arg Val Ser Gly Thr His Phe Pro Ile Arg Pro Leu
            500                 505                 510

Ala Trp Tyr Arg Gln Ala Pro Gly Lys Glu Arg Glu Leu Val Ala Ser
        515                 520                 525

Ile Ser Ala Gly His Val Thr Asn Tyr Asn Val Ala Val Glu Gly Arg
    530                 535                 540

Phe Ile Ile Ser Arg Asp Asn Tyr Lys Asp Thr Val Tyr Leu Lys Met
545                 550                 555                 560

Asn Asn Val Lys Pro Glu Asp Thr Ala Val Tyr Leu Cys Asn Leu Gly

                565                 570                 575

Pro Ala Thr Met Ser Thr Asp Phe Arg Pro Thr Asp Phe Trp Gly Gln
            580                 585                 590

Gly Thr Gln Val Thr Val Ser Ser Gly Gly Gly Gly Ala Leu Gln Phe
        595                 600                 605

Ser Gln Ala Gly Ala Ser Asp Ile Arg Asp Gln Ser Arg Asn Trp Leu
    610                 615                 620

Pro Gly Pro Cys Tyr Arg Gln Gln Arg Val Ser Lys Thr Ser Ala Asp
625                 630                 635                 640

Asn Asn Asn Ser Glu Tyr Ser Trp Thr Gly Ala Thr Lys Tyr His Leu
                645                 650                 655

Asn Gly Arg Asp Ser Leu Val Asn Pro Gly Pro Ala Met Ala Ser His
            660                 665                 670

Lys Asp Asp Glu Glu Lys Phe Phe Pro Gln Ser Gly Val Leu Ile Phe
        675                 680                 685

Gly Lys Gln Gly Ser Glu Lys Thr Asn Val Asp Ile Glu Lys Val Met
    690                 695                 700

Ile Thr Asp Glu Glu Glu Ile Arg Thr Thr Asn Pro Val Ala Thr Glu
705                 710                 715                 720

Gln Tyr Gly Ser Val Ser Thr Asn Leu Gln Ala Gly Asn Ala Gln Ala
                725                 730                 735

Ala Thr Ala Asp Val Asn Thr Gln Gly Val Leu Pro Gly Met Val Trp
            740                 745                 750

Gln Asp Arg Asp Val Tyr Leu Gln Gly Pro Ile Trp Ala Lys Ile Pro
        755                 760                 765

His Thr Asp Gly His Phe His Pro Ser Pro Leu Met Gly Gly Phe Gly
    770                 775                 780

Leu Lys His Pro Pro Pro Gln Ile Leu Ile Lys Asn Thr Pro Val Pro
785                 790                 795                 800

Ala Asn Pro Ser Thr Thr Phe Ser Ala Ala Lys Phe Ala Ser Phe Ile
                805                 810                 815

Thr Gln Tyr Ser Thr Gly Gln Val Ser Val Glu Ile Glu Trp Glu Leu
            820                 825                 830

Gln Lys Glu Asn Ser Lys Arg Trp Asn Pro Glu Ile Gln Tyr Thr Ser
        835                 840                 845

Asn Tyr Asn Lys Ser Val Asn Val Asp Phe Thr Val Asp Thr Asn Gly
    850                 855                 860

Val Tyr Ser Glu Pro Arg Pro Ile Gly Thr Arg Tyr Leu Thr Arg Asn
865                 870                 875                 880

Leu

<210>  4
<211>  300
<212>  PRT
<213>  Artificial Sequence

<220>
<223>  bispecific-nanobody adaptors

<400>  4

Met Ser Glu Val Gln Leu Val Glu Ser Gly Gly Lys Leu Val Gln Ala
1               5                   10                  15

Gly Gly Ser Leu Arg Leu Ser Cys Ser Ala Ser Gly Arg Thr Phe Ser
            20                  25                  30

Phe Ser Thr Ser Thr Met Gly Trp Phe Arg Gln Ala Pro Gly Lys Glu
        35                  40                  45

Leu Glu Phe Val Ala Ala Ile Asp Trp Ser Asp Phe Asn Thr Tyr Tyr
    50                  55                  60

Ala Asp Ser Val Lys Gly Arg Phe Thr Ile Ser Arg His Asn Pro Arg
65                  70                  75                  80

Asn Ser Val Tyr Leu Gln Leu Asn Ser Leu Lys Pro Glu Asp Thr Ala
                85                  90                  95

Val Tyr Tyr Cys Ala Ala His Ser Glu Thr Arg Gly Gly Thr Arg Tyr
            100                 105                 110

Phe Asp Arg Pro Ser Leu Tyr Asn Tyr Trp Gly Gln Gly Thr Gln Val
        115                 120                 125

Thr Val Ser Ser Gly Gly Gly Gly Ser Gly Gly Gly Gly Ser Gly Gly

130                 135                 140

Gly Gly Ser Gly Gly Gly Gly Ser Gly Gly Gly Gly Asn Met Ser Gln
145                 150                 155                 160

Val Gln Leu Val Glu Ser Gly Gly Gly Leu Val Gln Ala Gly Gly Ser
                165                 170                 175

Leu Arg Leu Ser Cys Ala Ala Ser Gly Arg Thr Phe Ser Asn Ser Ala
            180                 185                 190

Met Gly Trp Phe Arg Gln Ala Pro Gly Met Glu Arg Glu Phe Leu Ala
        195                 200                 205

Val Ile Ser Trp Ser Gly Gly Asn Thr Lys Tyr Ala Asp Ser Val Lys
    210                 215                 220

Gly Arg Phe Thr Ile Ser Arg Asp Asn Ala Lys Asn Thr Val Tyr Leu
225                 230                 235                 240

Gln Met Asn Ser Leu Lys Pro Glu Asp Thr Ala Val Tyr Tyr Cys Ala
                245                 250                 255

Ala Asp Thr Gly Leu Gly Pro Trp Tyr Asp Tyr Trp Gly Gln Gly Thr
            260                 265                 270

Gln Val Thr Val Ser Ser Ala Ala Ala His His His His His His Gly
        275                 280                 285

Ala Ala Glu Gln Lys Leu Ile Ser Glu Glu Asp Leu
    290                 295                 300

<210>  5
<211>  318
<212>  PRT
<213>  Artificial Sequence

<220>
<223>  bispecific-nanobody adaptors

<400>  5

Met Ser Glu Val Gln Leu Val Glu Ser Gly Gly Lys Leu Val Gln Ala
1               5                   10                  15

Gly Gly Ser Leu Arg Leu Ser Cys Ser Ala Ser Gly Arg Thr Phe Ser
            20                  25                  30

Phe Ser Thr Ser Thr Met Gly Trp Phe Arg Gln Ala Pro Gly Lys Glu
        35                  40                  45

Leu Glu Phe Val Ala Ala Ile Asp Trp Ser Asp Phe Asn Thr Tyr Tyr
    50                  55                  60

Ala Asp Ser Val Lys Gly Arg Phe Thr Ile Ser Arg His Asn Pro Arg
65                  70                  75                  80

Asn Ser Val Tyr Leu Gln Leu Asn Ser Leu Lys Pro Glu Asp Thr Ala
                85                  90                  95

Val Tyr Tyr Cys Ala Ala His Ser Glu Thr Arg Gly Gly Thr Arg Tyr
            100                 105                 110

Phe Asp Arg Pro Ser Leu Tyr Asn Tyr Trp Gly Gln Gly Thr Gln Val
        115                 120                 125

Thr Val Ser Ser Gly Gly Gly Gly Ser Gly Gly Gly Gly Ser Gly Gly
    130                 135                 140

Gly Gly Ser Gly Gly Gly Gly Ser Gly Gly Gly Gly Ser Met Ala Gln
145                 150                 155                 160

Val Gln Leu Val Glu Ser Gly Gly Gly Leu Val Gln Ala Gly Gly Ser
                165                 170                 175

Leu Arg Leu Ser Cys Ala Ala Ser Gly Arg Thr Asp Gly Met Tyr Ala
            180                 185                 190

Met Gly Trp Phe Arg Glu Ala Pro Gly Lys Glu Arg Glu Phe Val Ala
        195                 200                 205

Val Gln Asp Leu Thr Ala Ser Asn Thr Tyr Tyr Ser Ser Ala Val Lys
    210                 215                 220

Gly Arg Phe Thr Ile Ser Arg Asp Asn Ala Lys Asn Thr Ala Tyr Leu
225                 230                 235                 240

Gln Met Asn Arg Leu Lys Ser Glu Asp Thr Ala Val Tyr Tyr Cys Ala
                245                 250                 255

Ala Gly Pro Thr Leu Met Ser Ser Tyr Gly Asn Ser Ala Arg Asp Tyr
            260                 265                 270

Asp Tyr Trp Gly Gln Gly Thr Arg Val Thr Val Ser Ser Leu Glu Glu
        275                 280                 285

Pro Lys Thr Pro Lys Pro Gln Pro Ala Ala Ala His His His His His

290                 295                 300

His Gly Ala Ala Glu Gln Lys Leu Ile Ser Glu Glu Asp Leu
305                 310                 315

<210>  6
<211>  286
<212>  PRT
<213>  Artificial Sequence

<220>
<223>  bispecific-nanobody adaptors

<400>  6

Met Ser Asp Val Gln Leu Gln Ala Ser Gly Gly Gly Ser Val Gln Ala
1               5                   10                  15

Gly Gly Ser Leu Thr Leu Ser Cys Arg Tyr Ser Gly Gly Phe Val Phe
            20                  25                  30

Arg Val Ser Ser Met Gly Trp Tyr Arg Gln Ala Pro Gly Asn Gln Arg
        35                  40                  45

Glu Leu Ile Ala Thr Ile Thr Val Gly Gly Lys Thr Asn Tyr Lys Asp
    50                  55                  60

Ser Val Gln Gly Arg Phe Ile Ile Ser Arg Asn Asp Thr Thr Val Thr
65                  70                  75                  80

Leu Gln Met Asn Arg Leu Gln Pro Glu Asp Thr Ala Val Tyr Tyr Cys
                85                  90                  95

Asn Leu Ala Ser Thr Ala Val Gly Ala Asp Thr Trp Gly Gln Gly Thr
            100                 105                 110

Arg Val Thr Val Ser Ser Gly Gly Gly Gly Ser Gly Gly Gly Gly Ser
        115                 120                 125

Gly Gly Gly Gly Ser Gly Gly Gly Gly Ser Gly Gly Gly Gly Asn Met
    130                 135                 140

Ser Gln Val Gln Leu Val Glu Ser Gly Gly Gly Leu Val Gln Ala Gly
145                 150                 155                 160

Gly Ser Leu Arg Leu Ser Cys Ala Ala Ser Gly Arg Thr Phe Ser Asn
                165                 170                 175

Ser Ala Met Gly Trp Phe Arg Gln Ala Pro Gly Met Glu Arg Glu Phe
            180                 185                 190

Leu Ala Val Ile Ser Trp Ser Gly Gly Asn Thr Lys Tyr Ala Asp Ser
        195                 200                 205

Val Lys Gly Arg Phe Thr Ile Ser Arg Asp Asn Ala Lys Asn Thr Val
    210                 215                 220

Tyr Leu Gln Met Asn Ser Leu Lys Pro Glu Asp Thr Ala Val Tyr Tyr
225                 230                 235                 240

Cys Ala Ala Asp Thr Gly Leu Gly Pro Trp Tyr Asp Tyr Trp Gly Gln
                245                 250                 255

Gly Thr Gln Val Thr Val Ser Ser Ala Ala Ala His His His His His
            260                 265                 270

His Gly Ala Ala Glu Gln Lys Leu Ile Ser Glu Glu Asp Leu
        275                 280                 285

<210> 7
<211> 304
<212> PRT
<213> Artificial Sequence

<220>
<223> bispecific-nanobody adaptors

<400> 7

Met Ser Asp Val Gln Leu Gln Ala Ser Gly Gly Gly Ser Val Gln Ala
1               5                   10                  15

Gly Gly Ser Leu Thr Leu Ser Cys Arg Tyr Ser Gly Gly Phe Val Phe
            20                  25                  30

Arg Val Ser Ser Met Gly Trp Tyr Arg Gln Ala Pro Gly Asn Gln Arg
        35                  40                  45

Glu Leu Ile Ala Thr Ile Thr Val Gly Gly Lys Thr Asn Tyr Lys Asp
    50                  55                  60

Ser Val Gln Gly Arg Phe Ile Ile Ser Arg Asn Asp Thr Thr Val Thr
65                  70                  75                  80

Leu Gln Met Asn Arg Leu Gln Pro Glu Asp Thr Ala Val Tyr Tyr Cys
                85                  90                  95

Asn Leu Ala Ser Thr Ala Val Gly Ala Asp Thr Trp Gly Gln Gly Thr
            100                 105                 110

Arg Val Thr Val Ser Ser Gly Gly Gly Gly Ser Gly Gly Gly Gly Ser
        115                 120                 125

Gly Gly Gly Gly Ser Gly Gly Gly Gly Ser Gly Gly Gly Gly Ser Met
    130                 135                 140

Ala Gln Val Gln Leu Val Glu Ser Gly Gly Gly Leu Val Gln Ala Gly
145                 150                 155                 160

Gly Ser Leu Arg Leu Ser Cys Ala Ala Ser Gly Arg Thr Asp Gly Met
                165                 170                 175

Tyr Ala Met Gly Trp Phe Arg Glu Ala Pro Gly Lys Glu Arg Glu Phe
            180                 185                 190

Val Ala Val Gln Asp Leu Thr Ala Ser Asn Thr Tyr Tyr Ser Ser Ala
        195                 200                 205

Val Lys Gly Arg Phe Thr Ile Ser Arg Asp Asn Ala Lys Asn Thr Ala
    210                 215                 220

Tyr Leu Gln Met Asn Arg Leu Lys Ser Glu Asp Thr Ala Val Tyr Tyr
225                 230                 235                 240

Cys Ala Ala Gly Pro Thr Leu Met Ser Ser Tyr Gly Asn Ser Ala Arg
                245                 250                 255

Asp Tyr Asp Tyr Trp Gly Gln Gly Thr Arg Val Thr Val Ser Ser Leu
            260                 265                 270

Glu Glu Pro Lys Thr Pro Lys Pro Gln Pro Ala Ala Ala His His His
        275                 280                 285

His His His Gly Ala Ala Glu Gln Lys Leu Ile Ser Glu Glu Asp Leu
    290                 295                 300

<210>  8
<211>  292
<212>  PRT
<213>  Artificial Sequence

<220>
<223>  bispecific-nanobody adaptors

<400>  8

Met Ala Gln Val Gln Leu Gln Gln Ser Gly Gly Gly Leu Val Gln Ala
1               5                   10                  15

Gly Gly Ser Leu Thr Leu Ser Cys Arg Val Ser Gly Thr His Phe Pro
            20                  25                  30
Ile Arg Pro Leu Ala Trp Tyr Arg Gln Ala Pro Gly Lys Glu Arg Glu
        35                  40                  45
Leu Val Ala Ser Ile Ser Ala Gly His Val Thr Asn Tyr Asn Val Ala
    50                  55                  60
Val Glu Gly Arg Phe Ile Ile Ser Arg Asp Asn Tyr Lys Asp Thr Val
65                  70                  75                  80
Tyr Leu Lys Met Asn Asn Val Lys Pro Glu Asp Thr Ala Val Tyr Leu
                85                  90                  95
Cys Asn Leu Gly Pro Ala Thr Met Ser Thr Asp Phe Arg Pro Thr Asp
            100                 105                 110
Phe Trp Gly Gln Gly Thr Gln Val Thr Val Ser Ser Gly Gly Gly Gly
        115                 120                 125
Ser Gly Gly Gly Gly Ser Gly Gly Gly Gly Ser Gly Gly Gly Gly Ser
    130                 135                 140
Gly Gly Gly Gly Asn Met Ser Gln Val Gln Leu Val Glu Ser Gly Gly
145                 150                 155                 160
Gly Leu Val Gln Ala Gly Gly Ser Leu Arg Leu Ser Cys Ala Ala Ser
                165                 170                 175
Gly Arg Thr Phe Ser Asn Ser Ala Met Gly Trp Phe Arg Gln Ala Pro
            180                 185                 190
Gly Met Glu Arg Glu Phe Leu Ala Val Ile Ser Trp Ser Gly Gly Asn
        195                 200                 205
Thr Lys Tyr Ala Asp Ser Val Lys Gly Arg Phe Thr Ile Ser Arg Asp
    210                 215                 220
Asn Ala Lys Asn Thr Val Tyr Leu Gln Met Asn Ser Leu Lys Pro Glu
225                 230                 235                 240
Asp Thr Ala Val Tyr Tyr Cys Ala Ala Asp Thr Gly Leu Gly Pro Trp
                245                 250                 255
Tyr Asp Tyr Trp Gly Gln Gly Thr Gln Val Thr Val Ser Ser Ala Ala
            260                 265                 270

Ala His His His His His His Gly Ala Ala Glu Gln Lys Leu Ile Ser
        275                 280                 285

Glu Glu Asp Leu
    290

<210>  9
<211>  310
<212>  PRT
<213>  Artificial Sequence

<220>
<223>  bispecific-nanobody adaptors

<400>  9

Met Ala Gln Val Gln Leu Gln Gln Ser Gly Gly Gly Leu Val Gln Ala
1               5                   10                  15

Gly Gly Ser Leu Thr Leu Ser Cys Arg Val Ser Gly Thr His Phe Pro
            20                  25                  30

Ile Arg Pro Leu Ala Trp Tyr Arg Gln Ala Pro Gly Lys Glu Arg Glu
        35                  40                  45

Leu Val Ala Ser Ile Ser Ala Gly His Val Thr Asn Tyr Asn Val Ala
    50                  55                  60

Val Glu Gly Arg Phe Ile Ile Ser Arg Asp Asn Tyr Lys Asp Thr Val
65                  70                  75                  80

Tyr Leu Lys Met Asn Asn Val Lys Pro Glu Asp Thr Ala Val Tyr Leu
                85                  90                  95

Cys Asn Leu Gly Pro Ala Thr Met Ser Thr Asp Phe Arg Pro Thr Asp
            100                 105                 110

Phe Trp Gly Gln Gly Thr Gln Val Thr Val Ser Ser Gly Gly Gly Gly
        115                 120                 125

Ser Gly Gly Gly Gly Ser Gly Gly Gly Gly Ser Gly Gly Gly Gly Ser
    130                 135                 140

Gly Gly Gly Gly Ser Met Ala Gln Val Gln Leu Val Glu Ser Gly Gly
145                 150                 155                 160

Gly Leu Val Gln Ala Gly Gly Ser Leu Arg Leu Ser Cys Ala Ala Ser
                165                 170                 175

Gly Arg Thr Asp Gly Met Tyr Ala Met Gly Trp Phe Arg Glu Ala Pro
            180                 185                 190

Gly Lys Glu Arg Glu Phe Val Ala Val Gln Asp Leu Thr Ala Ser Asn
        195                 200                 205

Thr Tyr Tyr Ser Ser Ala Val Lys Gly Arg Phe Thr Ile Ser Arg Asp
    210                 215                 220

Asn Ala Lys Asn Thr Ala Tyr Leu Gln Met Asn Arg Leu Lys Ser Glu
225                 230                 235                 240

Asp Thr Ala Val Tyr Tyr Cys Ala Ala Gly Pro Thr Leu Met Ser Ser
                245                 250                 255

Tyr Gly Asn Ser Ala Arg Asp Tyr Asp Tyr Trp Gly Gln Gly Thr Arg
            260                 265                 270

Val Thr Val Ser Ser Leu Glu Glu Pro Lys Thr Pro Lys Pro Gln Pro
        275                 280                 285

Ala Ala Ala His His His His His His Gly Ala Ala Glu Gln Lys Leu
    290                 295                 300

Ile Ser Glu Glu Asp Leu
305                 310

<210>  10
<211>  403
<212>  PRT
<213>  Artificial Sequence

<220>
<223>  bispecific-nanobody adaptors

<400>  10

Met Ser Asp Val Gln Leu Gln Ala Ser Gly Gly Gly Ser Val Gln Ala
1               5                   10                  15

Gly Gly Ser Leu Thr Leu Ser Cys Arg Tyr Ser Gly Gly Phe Val Phe
            20                  25                  30

Arg Val Ser Ser Met Gly Trp Tyr Arg Gln Ala Pro Gly Asn Gln Arg
        35                  40                  45

Glu Leu Ile Ala Thr Ile Thr Val Gly Gly Lys Thr Asn Tyr Lys Asp
    50                  55                  60

Ser Val Gln Gly Arg Phe Ile Ile Ser Arg Asn Asp Thr Thr Val Thr

65                  70                  75                  80

Leu Gln Met Asn Arg Leu Gln Pro Glu Asp Thr Ala Val Tyr Tyr Cys
                85                  90                  95

Asn Leu Ala Ser Thr Ala Val Gly Ala Asp Thr Trp Gly Gln Gly Thr
            100                 105                 110

Arg Val Thr Val Ser Ser Gly Gly Gly Gly Ser Gly Gly Gly Gly Ser
        115                 120                 125

Gly Gly Gly Gly Ser Gly Gly Gly Gly Ser Met Ser Asp Val Gln Leu
    130                 135                 140

Gln Glu Ser Gly Pro Asp Leu Val Lys Pro Ser Gln Ser Leu Ser Leu
145                 150                 155                 160

Thr Cys Thr Val Thr Gly Tyr Ser Ile Thr Ser Gly Tyr Thr Trp His
                165                 170                 175

Trp Ile Arg Gln Phe Pro Gly Asn Lys Gln Glu Trp Met Gly Tyr Ile
            180                 185                 190

His Phe Ser Gly Tyr Thr Asn Tyr Asn Pro Ser Leu Lys Ser Arg Val
        195                 200                 205

Ser Ile Thr Arg Asp Thr Ser Lys Asn Gln Phe Phe Leu His Leu Asn
    210                 215                 220

Ser Val Thr Thr Glu Asp Thr Ala Thr Tyr Tyr Cys Ala Arg Gly Asp
225                 230                 235                 240

Tyr Gly Tyr Glu Trp Phe Thr Tyr Trp Gly Gln Gly Thr Leu Val Thr
                245                 250                 255

Val Leu Glu Gly Gly Gly Gly Ser Gly Gly Gly Gly Ser Gly Gly Gly
            260                 265                 270

Gly Ser Asp Ile Gln Met Thr Gln Ser Ser Ser Ser Phe Ser Val Ser
        275                 280                 285

Leu Gly Asp Arg Val Thr Ile Thr Cys Lys Ala Ser Glu Asp Ile His
    290                 295                 300

Asn Arg Leu Ala Trp Tyr Lys Gln Lys Pro Gly Asn Ala Pro Arg Leu
305                 310                 315                 320

Leu Ile Ser Gly Ala Thr Ser Leu Glu Thr Gly Val Pro Ser Arg Phe
                325                 330                 335

Ser Gly Ser Gly Ser Gly Lys Asp Tyr Thr Leu Ser Ile Thr Ser Leu
            340                 345                 350

Gln Asn Glu Asp Val Ala Thr Tyr Tyr Cys Gln Gln Tyr Trp Ile Gly
        355                 360                 365

Pro Phe Thr Phe Gly Ser Gly Thr Lys Leu Glu Ile Lys Ala Ala Ala
    370                 375                 380

His His His His His His Gly Ala Ala Glu Gln Lys Leu Ile Ser Glu
385                 390                 395                 400

Glu Asp Leu

<210>  11
<211>  122
<212>  PRT
<213>  Artificial Sequence

<220>
<223>  AAV nanobody

<400>  11

Gln Val Gln Leu Val Glu Ser Gly Gly Gly Leu Ala Pro Ala Gly Gly
1               5                   10                  15

Ser Leu Arg Leu Ser Cys Ala Ala Ser Gly Arg Thr Phe Ser Ser Tyr
            20                  25                  30

Ala Met Gly Trp Phe Arg Gln Ala Pro Gly Lys Glu Arg Glu Phe Val
        35                  40                  45

Ala Thr Ile Asn Trp Ser Asp Asn Ser Thr Tyr Tyr Val Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys

115                 120

<210>  12
<211>  122
<212>  PRT
<213>  Artificial Sequence

<220>
<223>  AAV nanobody

<400>  12

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Asn Ser
            20                  25                  30

Ala Met Gly Trp Phe Arg Gln Ala Pro Gly Met Glu Arg Glu Phe Leu
        35                  40                  45

Ala Val Ile Ser Trp Ser Gly Gly Asn Thr Lys Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  13
<211>  122
<212>  PRT
<213>  Artificial Sequence

<220>
<223>  AAV nanobody

<400>  13

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Asn Phe Ser Asn Ser
            20                  25                  30

Ala Met Gly Trp Phe Arg Gln Ala Pro Gly Thr Glu Arg Glu Phe Val
        35                  40                  45

Ala Gly Ile Ser Trp Ser Gly Gly Ser Thr Tyr Tyr Ala Asp Ser Val
    50                  55                  60

Lys Gly Arg Phe Thr Val Ser Arg Asp Asn Ala Lys Asn Thr Val Tyr
65                  70                  75                  80

Leu Gln Met Lys Ser Leu Lys Pro Glu Asp Thr Ala Val Tyr Tyr Cys
                85                  90                  95

Ala Ala Asp Thr Gly Leu Tyr Thr Trp Tyr Asp Tyr Trp Gly Gln Gly
            100                 105                 110

Thr Gln Val Thr Val Ser Ser Glu Pro Lys
        115                 120

<210>  14
<211>  122
<212>  PRT
<213>  Artificial Sequence

<220>
<223>  AAV nanobody

<400>  14

Gln Val Gln Leu Val Glu Ser Gly Gly Gly Leu Val Gln Ala Gly Asp
1               5                   10                  15

Ser Leu Arg Leu Ser Cys Thr Ala Ser Gly Arg Thr Phe Ser Asn Ser
            20                  25                  30

Ala Met Ala Trp Phe Arg Gln Ala Pro Gly Met Glu Arg Glu Phe Leu
        35                  40                  45

Ala Val Ile Ser Trp Ser Gly Asn Tyr Thr Lys Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys

115                 120

<210>  15
<211>  122
<212>  PRT
<213>  Artificial Sequence

<220>
<223>  AAV nanobody

<400>  15

Gln Val Gln Leu Val Glu Ser Gly Gly Gly Val Val Gln Ala Gly Gly
1               5                   10                  15

Ser Leu Arg Leu Ser Cys Ala Ala Ser Gly Arg Thr Phe Ser Lys Ser
            20                  25                  30

Thr Met Gly Trp Phe Arg Leu Ala Pro Gly Lys Glu Arg Glu Leu Val
        35                  40                  45

Ala Ala Ile Ser Trp Ser Gly Asp Asn Thr Asn Tyr Ala Asp Ser Val
    50                  55                  60

Lys Gly Arg Phe Ser Ile Ser Arg Asp Asn Ala Lys Asn Thr Val Tyr
65                  70                  75                  80

Leu Gln Met Asn Ser Leu Lys Ala Glu Asp Thr Ala Val Tyr Ser Cys
                85                  90                  95

Ala Ala Glu Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  16
<211>  137
<212>  PRT
<213>  Artificial Sequence

<220>
<223>  AAV nanobody

<400>  16

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp
            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135

<210>  17
<211>  137
<212>  PRT
<213>  Artificial Sequence

<220>
<223>  AAV nanobody

<400>  17

Glu Val Lys Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp

            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135

<210> 18
<211> 137
<212> PRT
<213> Artificial Sequence

<220>
<223> AAV nanobody

<400> 18

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp
            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135

<210> 19
<211> 25
<212> PRT
<213> Artificial Sequence

<220>

<223>  (GGGGS)5 linker

<400>  19

Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25

<210>  20
<211>  122
<212>  PRT
<213>  Artificial Sequence

<220>
<223>  AAV nanobody

<400>  20

Gln Val Gln Leu Val Glu Ser Gly Gly Gly Leu Ala Pro Ala Gly Gly
1               5                   10                  15

Ser Leu Arg Leu Ser Cys Ala Ala Ser Gly Arg Thr Phe Ser Ser Tyr
            20                  25                  30

Ala Met Gly Trp Phe Arg Gln Ala Pro Gly Lys Glu Arg Glu Phe Val
        35                  40                  45

Ala Thr Ile Asn Trp Ser Asp Asn Ser Thr Tyr Tyr Val Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  21
<211>  122
<212>  PRT
<213>  Artificial Sequence

<220>
<223>  AAV nanobody

<400>  21

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Phe Ser Asn Ser
            20                  25                  30

Ala Met Gly Trp Phe Arg Gln Ala Pro Gly Met Glu Arg Glu Phe Leu
        35                  40                  45

Ala Val Ile Ser Trp Ser Gly Gly Asn Thr Lys Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  22
<211>  122
<212>  PRT
<213>  Artificial Sequence

<220>
<223>  AAV nanobody

<400>  22

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Asn Phe Ser Asn Ser
            20                  25                  30

Ala Met Gly Trp Phe Arg Gln Ala Pro Gly Thr Glu Arg Glu Phe Val
        35                  40                  45

Ala Gly Ile Ser Trp Ser Gly Gly Ser Thr Tyr Tyr Ala Asp Ser Val
    50                  55                  60

Lys Gly Arg Phe Thr Val Ser Arg Asp Asn Ala Lys Asn Thr Val Tyr
65                  70                  75                  80

Leu Gln Met Lys Ser Leu Lys Pro Glu Asp Thr Ala Val Tyr Tyr Cys
                85                  90                  95

Ala Ala Asp Thr Gly Leu Tyr Thr Trp Tyr Asp Tyr Trp Gly Gln Gly
            100                 105                 110

Thr Gln Val Thr Val Ser Ser Glu Pro Lys
        115                 120

<210>  23
<211>  122
<212>  PRT
<213>  Artificial Sequence

<220>
<223>  AAV nanobody

<400>  23

Gln Val Gln Leu Val Glu Ser Gly Gly Gly Leu Val Gln Ala Gly Asp
1               5                   10                  15

Ser Leu Arg Leu Ser Cys Thr Ala Ser Gly Arg Thr Phe Ser Asn Ser
            20                  25                  30

Ala Met Ala Trp Phe Arg Gln Ala Pro Gly Met Glu Arg Glu Phe Leu
        35                  40                  45

Ala Val Ile Ser Trp Ser Gly Asn Tyr Thr Lys Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  24
<211>  122
<212>  PRT
<213>  Artificial Sequence

<220>
<223>  AAV nanobody

<400>  24

Gln Val Gln Leu Val Glu Ser Gly Gly Gly Val Val Gln Ala Gly Gly
1               5                   10                  15

Ser Leu Arg Leu Ser Cys Ala Ala Ser Gly Arg Thr Phe Ser Lys Ser
            20                  25                  30

Thr Met Gly Trp Phe Arg Leu Ala Pro Gly Lys Glu Arg Glu Leu Val
        35                  40                  45

Ala Ala Ile Ser Trp Ser Gly Asp Asn Thr Asn Tyr Ala Asp Ser Val
    50                  55                  60

Lys Gly Arg Phe Ser Ile Ser Arg Asp Asn Ala Lys Asn Thr Val Tyr
65                  70                  75                  80

Leu Gln Met Asn Ser Leu Lys Ala Glu Asp Thr Ala Val Tyr Ser Cys
                85                  90                  95

Ala Ala Glu Thr Gly Leu Gly Pro Trp Tyr Asp Tyr Trp Gly Gln Gly
            100                 105                 110

Thr Gln Val Thr Val Ser Ser Glu Pro Lys
        115                 120

<210>  25
<211>  137
<212>  PRT
<213>  Artificial Sequence

<220>
<223>  AAV nanobody

<400>  25

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp
            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135

<210>  26
<211>  137
<212>  PRT
<213>  Artificial Sequence

<220>
<223>  AAV nanobody

<400>  26

Glu Val Lys Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp
            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135

<210>  27

<211>  137
<212>  PRT
<213>  Artificial Sequence

<220>
<223>  AAV nanobody

<400>  27

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Thr Asp Gly Met Tyr
            20                  25                  30

Ala Met Gly Trp Phe Arg Glu Ala Pro Gly Lys Glu Arg Glu Phe Val
        35                  40                  45

Ala Val Gln Asp Leu Thr Ala Ser Asn Thr Tyr Tyr Ser Ser Ala Val
    50                  55                  60

Lys Gly Arg Phe Thr Ile Ser Arg Asp Asn Ala Lys Asn Thr Ala Tyr
65                  70                  75                  80

Leu Gln Met Asn Arg Leu Lys Ser Glu Asp Thr Ala Val Tyr Tyr Cys
                85                  90                  95

Ala Ala Gly Pro Thr Leu Met Ser Ser Tyr Gly Asn Ser Ala Arg Asp
            100                 105                 110

Tyr Asp Tyr Trp Gly Gln Gly Thr Arg Val Thr Val Ser Ser Leu Glu
        115                 120                 125

Glu Pro Lys Thr Pro Lys Pro Gln Pro
    130                 135


<210>  28
<211>  10
<212>  PRT
<213>  Artificial Sequence

<220>
<223>  CDR3

<400>  28

Asp Thr Gly Leu Gly Pro Trp Tyr Asp Tyr
1               5                   10


<210>  29
<211>  10
<212>  PRT
<213>  Artificial Sequence

<220>
<223>  CDR3

<400>  29

Asp Thr Gly Leu Tyr Thr Trp Tyr Asp Tyr
1               5                   10


<210>  30
<211>  10
<212>  PRT
<213>  Artificial Sequence

<220>
<223>  CDR3

<400>  30

Glu Thr Gly Leu Gly Pro Trp Tyr Asp Tyr
1               5                   10


<210>  31
<211>  17
<212>  PRT
<213>  Artificial Sequence

<220>
<223>  CDR3

<400>  31

Gly Pro Thr Leu Met Ser Ser Tyr Gly Asn Ser Ala Arg Asp Tyr Asp
1               5                   10                  15


Tyr